# EUROPEAN PATENT APPLICATION

(11) **EP 2 669 276 A1**
(43) Date of publication of application: **04.12.2013**
(21) Application number: 12305601.2
(22) Date of filing: 31.05.2012
(51) Int. Cl.: C07D 295/15, C07C 229/04, A61K 31/4453, A61K 31/164, A61K 31/165, A61K 31/167

(54) **Ornithine- and lysine-derivatives for the treatment of pain**

(71) Applicant: Université de Strasbourg, 67000 Strasbourg (FR); Université Bordeaux Segalen, 33076 Bordeaux Cedex (FR); Centre National de la Recherche Scientifique, 75016 Paris (FR)
(72) Inventor: Bourguignon, Jean-jacques, 67400 Illkirch (FR); Bihel, Frédéric, 67640 Fegerscheim (FR); Schmitt, Martine, 67000 Strasbourg (FR); Simonin, Frédéric, 67190 Gresswiller (FR); Simonnet, Guy, 33200 Bordeaux (FR); Humbert, Jean-Paul, 67220 Bassemberg (FR); Schneider, Séverine, 67000 Strasbourg (FR); Laboureyras, Emilie, 33000 Bordeaux (FR)
(74) Representative: Tezier Herman, Béatrice

(57) **Abstract**

The invention relates to novel compounds, derivatives of ornithine or of lysine, to pharmaceutical compositions containing same and to the use of same in the treatment of pain.

## Description

The invention relates to novel compounds, derivatives of ornithine or of lysine, to pharmaceutical compositions containing same and to the use of same in the treatment of pain.

### Background of the invention

The treatment of pain, in particular chronic pain, is a major public health issue. The use of opiate analgesics (such as morphine or fentanyl) constitutes an effective treatment for acute pain. However, their repeated and prolonged use leads to a loss of effectiveness (tolerance), followed by hypersensitivity to pain (hyperalgesia), making such treatments complex and delicate for the treatment of chronic pain.

The invention describes a novel series of non-natural derivatives of ornithine and of lysine that have high affinity for neuropeptide FF (NPFF) receptors, in particular NPFF1 and NPFF2 receptors, which are involved in the modulation of nociceptive signals. In 2006 in PNAS (Simonin et al., PNAS (2006) 103, 2, 466-71), the dipeptide RF9 (referred to as N^{α}-adamantan-1-yl-L-Arg-L-Phe-NH₂ acetate in WO02/24192) was described as being the first nanomolar NPFF receptor antagonist. Administered *in vivo* in the rat, RF9 showed antihyperalgesic activity, reversing hyperalgesia induced by the repeated administration of opiate analgesics.

Opiate analgesics are at present the treatment of choice for moderate or severe pain. For many patients, notably those suffering from advanced cancer, the treatment of pain requires strong, repeated doses of opiates such as morphine or fentanyl. The clinical effectiveness and tolerability of such treatments are, however, qualified by two phenomena induced by the use of opiates. The first is the tolerance effect, which is characterized by a shortening of action duration and a reduction in analgesia intensity. The clinical result is a growing need to increase the doses of opiates in order to maintain the same analgesic effect, uncorrelated with a progression of the disease. The second problem, related to repeated administration of strong doses of opiates, is known as opioid-induced hyperalgesia (OIH). Indeed, prolonged administration of opiates leads to a paradoxical increase in pain, unrelated to the initial nociceptive stimulus.

It has been suggested that such hyperalgesia would be the cause of tolerance. Tolerance would indeed be apparent since the analgesic effect characteristic of each daily dose remains constant; it would thus be the development of hypersensitivity to pain which would give the impression of a decrease in the effects of the opiate. It would thus not be the opiate that would have lost its effectiveness, but the individual who would have become hypersensitive to pain.

These two phenomena have been widely documented in both animal and human studies. Overall, they have been observed after administration of all types of opiates, regardless of the routes of administration or the doses used.

Furthermore, the administration of high doses of opiates leads to a certain number of side effects such as nausea, constipation, sedation and respiratory deficiencies (e.g.: delayed respiratory depression).

Currently, several strategies for mitigating these opiate-induced effects of tolerance and hyperalgesia are under investigation:
1) One of the most commonly used clinical strategies consists of combining opiates with adjuvants such as anticonvulsants or antidepressants, particularly in the treatment of neuropathic pain. In spite of some effectiveness, these additives involve numerous side effects, notably cardiac risks.
2) The rotation of opiates is also used as an alternative strategy, supported by the fact that different opiates have different affinities for each receptor, and that tolerance develops independently for each receptor. However, very few results have been described, and this strategy is the subject of much discussion.
3) NMDA receptor antagonists are known to block calcium channels, which leads in man or animals to a reduction in opiate-induced hyperalgesia as well as to a delay in tolerance effects. However, the clinical use of ketamine as an NMDA receptor antagonist involves a broad spectrum of side effects in man, notably hallucinations.

Although a certain amount of success has been reported, no strategy at present effectively blocks the effects of hyperalgesia and tolerance related to the repeated use of opiates. Consequently, the search for alternative strategies is necessary, notably in the field of neuropathic or cancer pain. Indeed, in the context of these pathologies, the treatments currently used are relatively ineffective and involve the use of high doses of opiates, leading to many particularly disabling side effects. Consequently, a major therapeutic issue relates to the development of novel drugs that act on novel therapeutic targets involved in the modulation of pain.

Research is currently under way for therapies that improve the use of opioid analgesics in man, in particular during the long-term use or the single administration of high doses, as is the case during surgical procedures.

Among the anti-opiate systems responsible for the loss of effectiveness of opiate analgesics and the appearance of hyperalgesia, NPFF receptors appear to be relevant targets. The design of drugs that inhibit the action of these receptors will make it possible to restore the long-term effectiveness of opiate analgesics while preventing the appearance of opiate-induced hyperalgesia.

In that context, a first patent application published under the number WO02/24192 described Arg-Phe dipeptide derivatives which provided proof of this concept *in vivo.* In particular, a single administration of RF9 (0.5 mg/kg, i.p.) in the rat blocks hyperalgesia induced by administration of fentanyl, an opiate analgesic that acts as a µ receptor agonist and is typically used in a hospital setting.

### Summary of the invention

The present invention describes a family of non-natural derivatives of ornithine and of lysine whose therapeutic use could enable better treatment of postoperative pain or of chronic pain accompanying certain pathologies such as diabetes, cancer, inflammatory disease (rheumatoid arthritis, for example) or neuropathy. These types of pain are regarded as severe and particularly disabling.

The compounds of the present invention are non-natural amino acids that are powerful NPFF1 and/or NPFF2 receptor ligands. Certain compounds show selectivity for NPFF 1 or NPFF2.

More particularly, in the rat, an ornithine derivative according to the invention (a single 0.5 mg/kg dose, i.p., of compound 26) blocks hyperalgesia induced by administration of fentanyl. The selectivity of this compound for NPFF1 receptors, located in the supraspinal region, shows the involvement of these receptors in the control of OIH. The advantage of said derivative, as a representative of this novel family of NPFF receptor ligands, is due to the fact that, in contrast to dipeptides represented by RF9, this compound shows highly satisfactory *in vivo* activity after oral administration of a low dose of 1 mg/kg. Moreover, its effectiveness by oral route was confirmed in a dose-dependent manner.

Furthermore, the study of said compound shows that an NPFF receptor ligand has an intrinsic effect on hyperalgesia induced by inflammatory or neuropathic pain, notably by blocking latent pain sensitization that occurs after opiate administration and which is responsible for exaggerated hypersensitivity to a future nociceptive stimulus.

Thus, the present invention describes a novel type of NPFF receptor ligand compounds whose administration in a mammal, for example by oral or subcutaneous route, opposes hyperalgesic effects induced by administration of opiate analgesics, and also decreases, even blocks, latent pain hypersensitivity to a future nociceptive stimulus.

Latent sensitization to pain corresponds to a phenomenon observed after effective treatment of pain in which the individual does not return to an initial state (homeostasis) but actually becomes vulnerable to any new nociceptive stimulus. Any new tissue injury will thus result in exaggerated pain (hyperalgesia), in particular in terms of duration which may greatly exceed that of the injury. Similarly, it has been shown that sensitized animals no longer exhibit a non-nociceptive stress-induced analgesia response as classically described in "naive" animals, but rather exhibit an opposite response, *i.e.,* of stress-induced hyperalgesia. Increasingly long-lasting hyperalgesia occurs when this type of stress is repeated over time (Endogenous Opioids Released During Non-Nociceptive Environmental Stress Induce Latent Pain Sensitization Via a NMDA-Dependent Process. Pain. 2011 Oct;12(10):1069-79; Le Roy C, Laboureyras E, Gavello-Baudy S, Chateauraynaud J, Laulin JP, Simonnet G).

The therapeutic prospects envisaged consist notably of co-administration of these compounds with opiate analgesics in the context of the treatment of postoperative pain, but also for the treatment of severe chronic pain caused by inflammation, neuropathy, cancer, diabetes or drugs. Furthermore, the effect of the compounds according to the invention on hypersensitivity to pain makes it possible to also envisage the administration of said compounds alone in the context of the prophylactic treatment of pain.

One object of the invention thus relates to compounds of general formula (I), notably as drugs, and to a method for preparing same. The invention also relates to pharmaceutical compositions comprising the compounds according to the invention in a pharmaceutically acceptable carrier.

The compounds and the pharmaceutical compositions according to the invention are particularly suited for use in the treatment of pain. In particular, the compounds and compositions according to the invention decrease or block opiate-related hyperalgesia and/or tolerance or decrease or block exaggerated hypersensitivity to future nociceptive stimuli.

Thus, the compounds and the pharmaceutical compositions according to the invention may be used in the treatment of postoperative pain or of severe chronic pain caused by inflammation, neuropathy, cancer, diabetes or drugs.

The invention also describes a method for treating pain in a subject, comprising the administration to said patient of an effective quantity of a compound according to the invention.

### Brief description of the figures

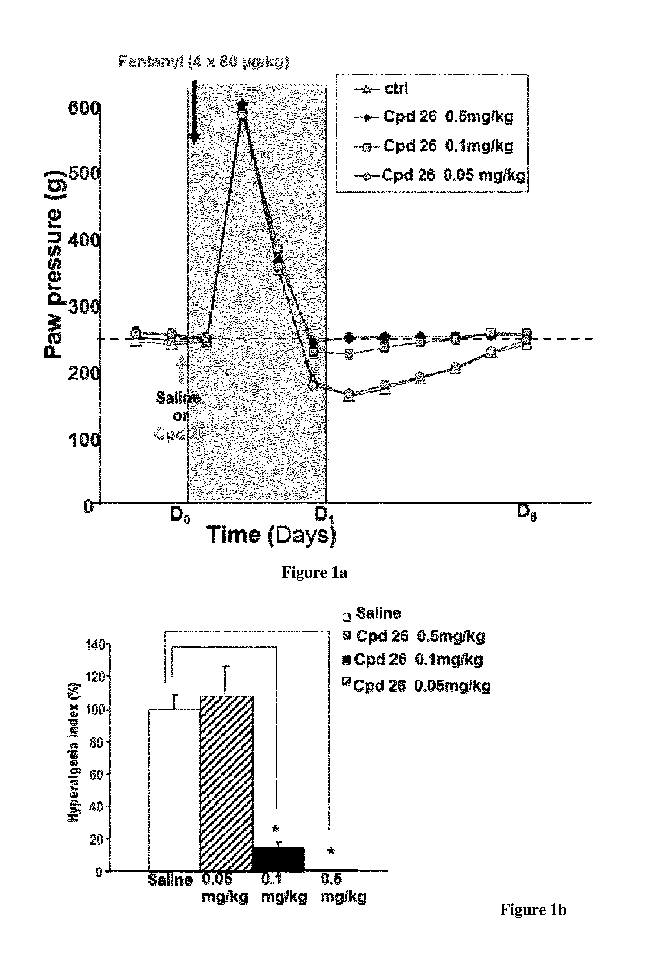

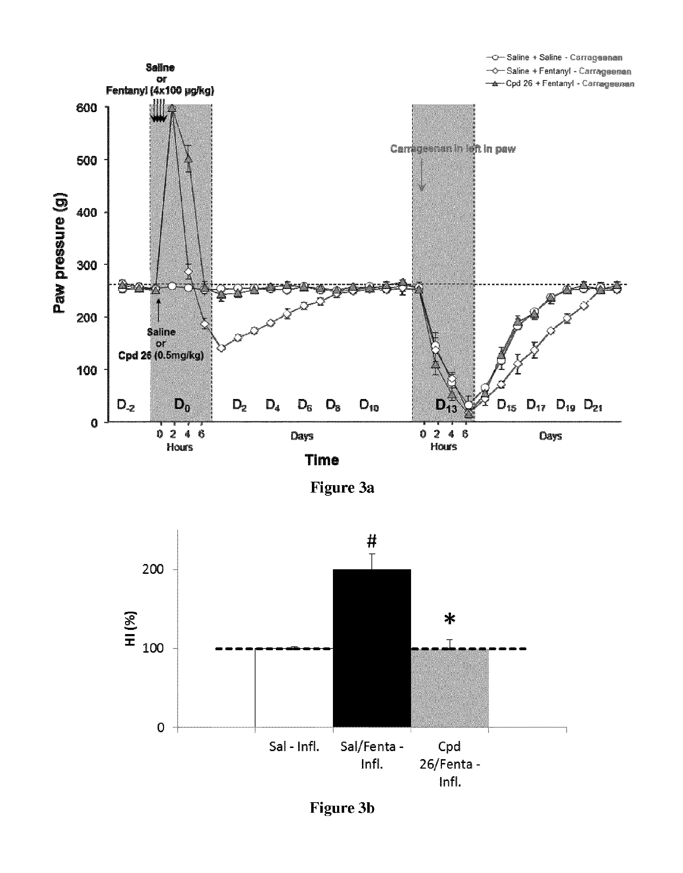

Figure 1: (a) Paw pressure vocalization test with fentanyl injections without administration of compound 26 (administration of a saline solution) 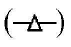 or with co-administration by subcutaneous injection of 0.5 mg/kg 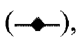 , 0,1mg/kg 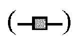 or 0.05mg/kg 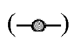 of compound 26 and (b) hyperalgesia index (HI) and statistical analysis of figure 1 (a).

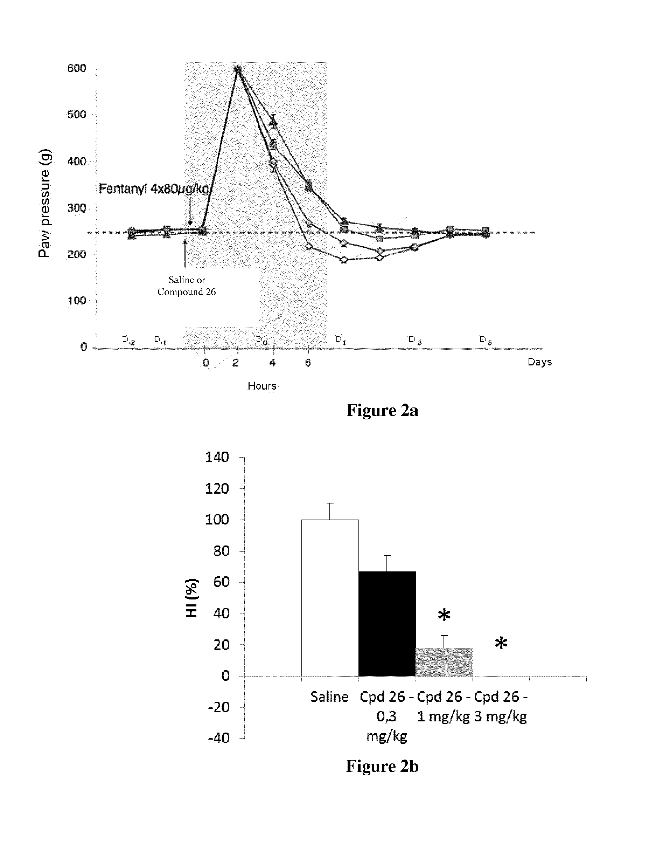

Figure 2: (a) Paw pressure vocalization test with fentanyl injections without administration of compound 26 (administration of a saline solution) 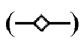 or with co-administration by oral route of 0.3 mg/kg 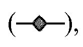 , 1mg/kg 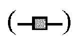 or 3mg/kg 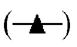 of compound 26 and (b) hyperalgesia index (HI) and statistical analysis of figure 2(a).
Figure 3:
   - (a) Paw pressure vocalization test on the left (inflamed) hind paw with administration of saline solution at day 0 (D0) 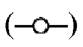 or with fentanyl injections without administration of compound 26 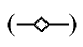 at D0 or with co-administration by subcutaneous injection of 0.5 mg/kg 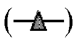 at D0 of compound 26 and (b) hyperalgesia index (HI) and statistical analysis of figure 3(a).
   - (c) Paw pressure vocalization test on the right (non-inflamed) hind paw with administration of saline solution at day 0 (D0) 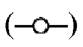 or with fentanyl injections without administration of compound 26 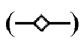 at D0 or with co-administration by subcutaneous injection of 0.5 mg/kg 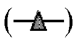 of compound 26 at D0 and (d) hyperalgesia index (HI) and statistical analysis of figure 3(a).

### Detailed description of the invention:

The compounds according to the invention have the following general formula (I): 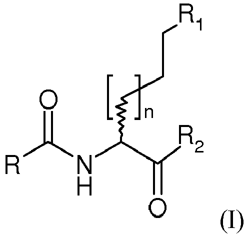 wherein:
- n is 1 or 2;
- R is a (C₁-C₄)alkyl, (C₂-C₄)alkenyl, (C₆-C₁₄)aryl, heteroaryl, aralkyl, heteroaralkyl or (C₃-C₁₀)cycloalkyl group, wherein said group is optionally substituted by at least one substituent selected from a halogen atom, a (C₁-C₄)alkyl, (C₁-C₄)alkoxy, thioalkyl, aryl, aralkyl or heteroaryl group, wherein said substituent group is optionally substituted by at least one group selected from: -COOH, halogen, (C₁-C₄)alkyl, (C₁-C₄)alkoxy, perhalogeno(C₁-C₄)alkyl, hydroxyl, NO₂ and NH₂;
- R₁ represents an amino group selected from:
   (i) 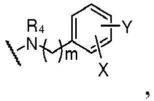 wherein m is 1, 2 or 3, R₄ is a hydrogen atom or a (C₁-C₄)alkyl group, X and Y independently represent a hydrogen atom, a halogen atom, a hydroxyl, a (C₁-C₄)alkoxy, a perhalogeno(C₁-C₄)alkyl group, an acetyl group (-COCH₃), or an amino group that is mono- or bi-substituted by an alkyl or heterocycle group, or X and Y may form together an -O-(CH₂)ₚ-O- group, wherein p is 1, 2 or 3;
   (ii) 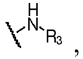 wherein R₃ is a hydrogen atom, a (C₁-C₄)alkyl, aralkyl or (C₅-C₈)cycloalkyl group;
   (iii) 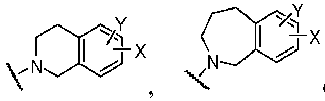 or 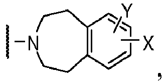 wherein X and Y independently represent a hydrogen atom, a halogen atom, a hydroxyl, a (C₁-C₄)alkyl, a (C₁-C₄)alkoxy, a perhalogeno(C₁-C₄)alkyl group, an acetyl group (-COCH₃), or an amino group that is mono- or bi-substituted by an alkyl or heterocycle group, or X and Y may form together an -O-(CH₂)ₚ-O- group, wherein p is 1, 2 or 3;
   (iv) 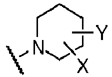 or 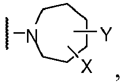 wherein X represents a hydrogen atom; a halogen atom; a hydroxyl group; an acetyl group (-COCH₃); an aryl; aryloxy; aralkyl; N-heterocycle; aryl-NH-; aralkyl-NH-; aroyl-NH; -NHCOCH₃ ;-NHCHO group; a 2-oxoindoline group; a -CO-R₅ group, wherein R₅ is an amino group that is mono- or bi-substituted by an alkyl or heterocycle group; a (C₁-C₄)alkyl group; a perhalogeno(C₁-C₄)alkyl group; a (C₁-C₄)alkoxy group; -NR₆COR₇, wherein R₆ is a hydrogen atom or a (C₁-C₄)alkyl group, and R₇ is a hydrogen atom, a (C₁-C₄)alkyl, aryl, or aralkyl group; a -ZR₈R₉ group, wherein Z is N or CH, R₈ is an aryl group and R₉ is a hydrogen atom, an aryl or (C₁-C₄)alkyl group; and Y is a hydrogen atom or a hydroxyl, (C₁-C₄)alkoxy, - NHCHO or NHCOCH₃ group;
   (v) 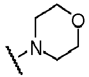 , 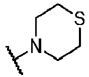 or 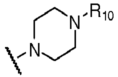 wherein R₁₀ is a hydrogen atom, a (C₁-C₄)alkyl group or a -CO-R₁₁ group wherein R₁₁ is a hydrogen atom or a (C₁-C₄)alkyl,
      aryl or heteroaryl group (such as 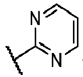 );
   (vi) 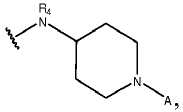 wherein R₄ is as defined above and A is a hydrogen atom or an alkyl or aralkyl group;
   (vii) 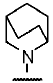 or 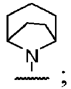
   (viii) 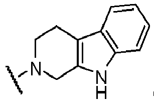 or 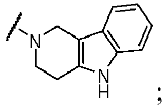
- R₂ represents an amino group selected from:
   (i) 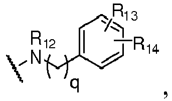 wherein q is 1, 2 or 3, R₁₂ is a hydrogen atom or a (C₁-C₄)alkyl group, R₁₃ is a hydrogen atom, a halogen atom, a (C₁-C₄)alkyl, a perhalogeno(C₁-C₄)alkyl group, (C₁-C₄)alkoxy group, an hydroxyl group, an acetyl group (-COCH₃), or an amino group that is mono- or bi-substituted by an alkyl or heterocycle group, R₁₄ is a hydrogen atom, a halogen atom, a (C₁-C₄)alkyl, a perhalogeno(C₁-C₄)alkyl group, a (C₁-C₄)alkoxy group or an amino group that is mono- or bi-substituted by an alkyl or heterocycle group, and R₁₃ and R₁₄ may form together an -O-(CH₂)ᵣ-O- group, wherein r is 1, 2 or 3;
   (ii) 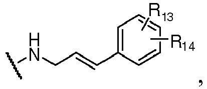 wherein R₁₃ and R₁₄ are as defined above;
   (iii) 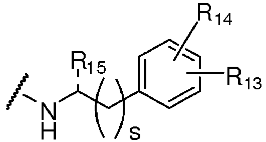 or 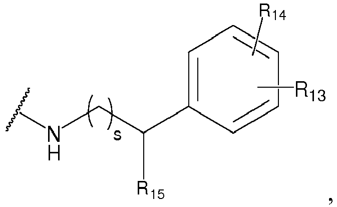 wherein R₁₃ and R₁₄ are as defined above, R₁₅ is a hydrogen atom or a (C₁-C₄)alkyl group, and s is 0 or 1;
   (iv) 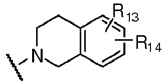 or 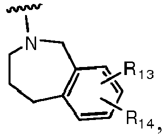 wherein R₁₃ and R₁₄ are as defined above;
   (v) 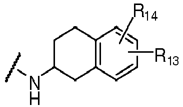 wherein R₁₃ and R₁₄ are as defined above; and
   (vi) 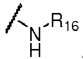 wherein R₁₆ is a hydrogen atom or a (C₁-C₄)alkyl group.
   (vii) 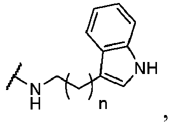 wherein n is 0 or 1.

According to the invention, the term "alkyl" refers to a linear or branched saturated hydrocarbon radical with 1 to 6 carbon atoms, such as methyl, ethyl, propyl, isopropyl, butyl, isobutyl, tert-butyl, pentyl, neopentyl or N-hexyl. C₁-C₄ groups are preferred.

According to the invention, the term "cycloalkyl" refers to a cyclic saturated hydrocarbon radical with 3 to 10 carbon atoms. Examples of cycloalkyls notably include cyclopropyl, cyclopentyl, cyclohexyl and adamantyl.

The term "heterocycle" refers to a cyclic saturated hydrocarbon radical with 3 to 10 carbon atoms comprising one or more cyclic heteroatoms, preferably 1 to 4 cyclic heteroatoms, wherein the heteroatoms are preferably selected from N, O, S and P. Notably, mention may be made of the group comprising piperidine, piperazine, homopiperidine and morpholine,

The term "alkenyl" refers to a linear or branched hydrocarbon radical with at least one ethylene unsaturation and with 2 to 6 carbon atoms, preferably 2 to 4 carbon atoms. Examples of alkenyls are notably 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl and isomeric forms of same (geometric isomers, *cis* and/or *trans* forms).

"Aryl" groups are monocyclic or bicyclic aromatic hydrocarbon systems with 6 to 14 carbon atoms. Among aryl groups, mention may be made of the group comprising phenyl, naphthyl, anthracenyl and fluorenyl. Preferably, the aryl groups are phenyl or naphthyl groups.

Heteroaryl groups are aryl groups comprising one or more cyclic heteroatoms, preferably 1 to 4 cyclic heteroatoms, wherein the heteroatoms are preferably selected from N, O, S and P. Among heteroaryl groups, mention may be made of the group comprising furan, pyridine, pyrimidine, thiophene, indole, imidazole, quinoline, isoquinoline, benzofuran, and benzimidazole.

An aromatic group is typically a group comprising 4 to 15 links including 0 to 4 heteroatoms, preferentially 4 to 12 links including 0 to 3 heteroatoms. An aromatic group may be monocyclic or polycyclic. Moreover, an aromatic group may comprise one or more aromatic rings condensed in a non-aromatic ring, such as hydroisoquinolines (such as 1,2,3,4-tetrahydroisoquinoline) or oxoindolines (such as 2-oxoindoline).

The term "aralkyl" (or "arylalkyl") refers to an aryl group as defined above bound to the remainder of the molecule by an alkyl group as defined above.

The term "heteroaralkyl" refers to a heteroaryl group as defined above bound to the remainder of the molecule by an alkyl group as defined above.

Alkoxy groups correspond to the alkyl groups defined above bound to the remainder of the molecule via an -O- (ether) bond. Aryloxy groups correspond to the aryl groups defined above bound to the remainder of the molecule via an -O- (ether) bond.

Thioalkyl groups correspond to the alkyl groups defined above bound to the remainder of the molecule via an -S- (thioether) bond.

The term "perhalogeno(C₁-C₄)alkyl" refers to a C₁-C₄ alkyl group substituted by atoms of halogen, notably such as the -CF₃ radical.

"Halogen" refers to an atom of fluorine, chlorine, bromine or iodine.

"Heteroatom" refers to an atom selected from O, N, P and S.

The compounds according to the invention also include enantiomers of same (pure or in mixtures, in particular racemic mixtures), geometric isomers of same, salts, hydrates and solvates of same, solid forms of same, as well as mixtures of said forms.

When the compounds according to the invention are in the forms of salts, they are preferably pharmaceutically acceptable salts. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Further examples of pharmaceutically acceptable inorganic or organic acid addition salts include the pharmaceutically acceptable salts listed in J. Pharm. Sci. 1977, 66, 2.

The compounds according to the invention are preferably compounds of formula (I) wherein R represents a group selected from:
(i) 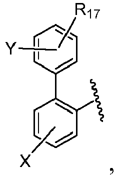 wherein R₁₇ is a hydrogen atom, a halogen atom or a (C₁-C₄)alkyl, perhalogeno(C₁-C₄)alkyl, (C₁-C₄)thioalkyl or (C₁-C₄)alkoxy group; X and Y independently represent a hydrogen atom, a halogen atom, a hydroxyl, a (C₁-C₄)alkoxy group, a perhalogeno(C₁-C₄)alkyl group or an amino group that is mono- or bi-substituted by an alkyl or heterocycle group, or X and Y may form together an -O-(CH₂)ₚ-O- group, wherein p is 1, 2 or 3;
(ii) 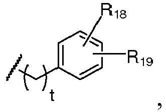 wherein t is 0, 1, 2, 3, 4, or 5 (preferably 0), R₁₈ is a hydrogen atom, a halogen atom, a hydroxyl, (C₁-C₄)alkyl, (C₁-C₄)alkoxy, (C₁-C₄)thioalkyl or perhalogeno(C₁-C₄)alkyl group, an amino group mono- or bi-substituted by an alkyl or heterocycle group, an NO₂, aryl, heteroaryl or aralkyl group wherein said aryl, heteroaryl or aralkyl group is optionally substituted by at least one group selected from: a halogen atom, a hydroxyl, (C₁-C₄)alkyl, (C₁-C₄)alkoxy, (C₁-C₄)thioalkyl, perhalogeno(C₁-C₄)alkyl or COOH group, R₁₉ is a hydrogen atom, a halogen atom, a hydroxyl, (C₁-C₄)alkyl, (C₁-C₄)alkoxy, (C₁-C₄)thioalkyl or perhalogeno(C₁-C₄)alkyl group, or an amino group mono- or bi-substituted by an alkyl or heterocycle group;
(iii) 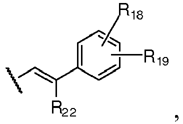 wherein R₂₂ is a hydrogen atom or a (C₁-C₄)alkyl group, and R₁₈ and R₁₉, independently, are as defined above,
(iv) 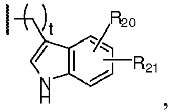 wherein t is 0, 1, 2, 3, 4, or 5, and R₂₀ is a hydrogen atom, a halogen atom, a (C₁-C₄)alkyl, perhalogeno(C₁-C₄)alkyl, (C₁-C₄)alkoxy; (C₁-C₄)thioalkyl or aryl group, or an amino group mono- or bi-substituted by an alkyl or heterocycle group, R₂₁ is a hydrogen atom, a halogen atom, a (C₁-C₄)alkyl, perhalogeno(C₁-C₄)alkyl, (C₁-C₄)alkoxy or (C₁-C₄)thioalkyl group, or an amino group mono- or bi-substituted by an alkyl or heterocycle group, wherein R₂₀ and R₂₁ together may also form an -O-(CH₂)ᵤ-O- ring attached to the phenyl group, wherein u is 1, 2 or 3;
(v) 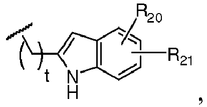 wherein t, R₂₀ and R₂₁ are as defined above;
(vi) 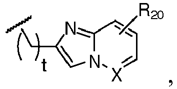 wherein t and R₂₀ are as defined above, and X is N or CH;
(vii) 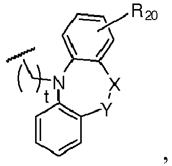 wherein t and R₂₀ are as defined above, X-Y is CH₂-CH₂, CO-CH₂, CO-N(alkyl) or CH=CH;
(viii) 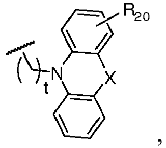 wherein t and R₂₀ are as defined above, X is S, O, NH, N(alkyl) or CH₂,
(ix) benzofuran, more specifically 1-benzofuran-2-yl, and
(x)

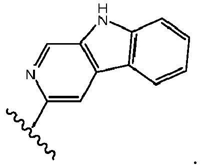

According to one variant of the invention, the compounds according to the invention are of formula (I) wherein R represents a group selected from:
- 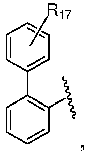 wherein R₁₇ is a hydrogen atom, a halogen atom, a (C₁-C₄)alkyl, perhalogeno(C₁-C₄)alkyl, (C₁-C₄)thioalkyl or (C₁-C₄)alkoxy group, preferably R₁₇ is a hydrogen atom or a (C₁-C₄)alkoxy group, in particular OCH₃;
- 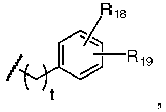 wherein t is 0, 1, 2, 3, 4, or 5 (preferably 0), R₁₈ is a hydrogen atom, a halogen atom or a hydroxyl or (C₁-C₄)alkoxy group, and R₁₉ is a hydrogen atom, a halogen atom or a hydroxyl or (C₁-C₄)alkoxy group;
- a cycloalkyl group, preferably an adamantyl ring: 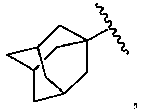 optionally substituted by -COOH, halogen, (C₁-C₄)alkyl, (C₁-C₄)alkoxy, perhalogeno(C₁-C₄)alkyl, hydroxyl, NO₂ or NH₂, in particular by at least one halogen atom or (C₁-C₄)alkyl or (C₁-C₄)alkoxy group;
- a heteroaryl group selected from a pyridine, furan, benzofuran, quinoline, indole and benzimidazole group, wherein said group is optionally substituted by at least one group selected from: -COOH, halogen, (C₁-C₄)alkyl, (C₁-C₄)alkoxy, perhalogeno(C₁-C₄)alkyl, hydroxyl, NO₂ and NH₂, in particular by at least one halogen atom or a (C₁-C₄)alkyl or (C₁-C₄)alkoxy group;
- 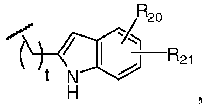 wherein t, R₂₀ and R₂₁ are as defined above, in particular t is 0, 1 or 2, and/or in particular R₂₀ and R₂₁, identical or different, are a hydrogen atom, a halogen atom or a (C₁-C₄)alkyl, perhalogeno(C₁-C₄)alkyl, or (C₁-C₄)alkoxy group;
- 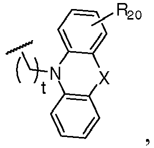 wherein t and R₂₀ are as defined above, X is S, O, NH, N(alkyl), or CH₂, in particular t is 1 or 2, and/or in particular R₂₀ is a hydrogen atom, a halogen atom or a (C₁-C₄)alkyl, perhalogeno(C₁-C₄)alkyl or (C₁-C₄)alkoxy group, and/or in particular X is S.

According to another variant of the invention, the compounds according to the invention are of formula (I) wherein R₁ represents an amino group selected from:
(i) 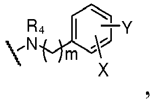 wherein m, R₄, X and Y are as defined above, in particular m is 1 or 2, R₄ is a hydrogen atom or a (C₁-C₄)alkyl group (such as methyl), X and Y independently represent a hydrogen atom, a halogen atom or a (C₁-C₄)alkoxy group (such as methoxy);
(ii) 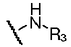 wherein R₃ is as defined above, in particular R₃ is a hydrogen atom or a (C₁-C₄)alkyl or (C₅-C₈)cycloalkyl group;
(iii) 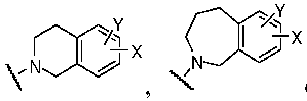 or 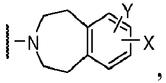 wherein X and Y are as defined above, in particular X and Y represent a hydrogen atom;
(iv) 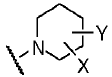 or 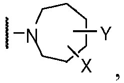 wherein X and Y are as defined above, in particular X represents a hydrogen atom, an aryl (such as phenyl), aralkyl (such as benzyl or phenethyl), N-piperidine or 2-oxoindoline group, and Y represents a hydrogen atom or a hydroxyl group;
(v) 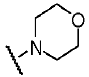 or 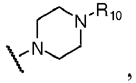 wherein R₁₀ is as defined above, in particular R₁₀ is a (C₁-C₄)alkyl group (such as methyl) or a -CO-R₁₁ group, wherein R₁₁ is a hydrogen atom or a (C₁-C₄)alkyl (such as methyl) or aryl group (such as phenyl);
(vi) 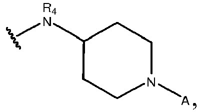 wherein R₄ and A are as defined above, in particular R₄ is a (C₁-C₄)alkyl group (such as methyl) and A is an aralkyl group (such as benzyl).

According to another variant of the invention, the compounds according to the invention are of formula (I) wherein R₂ represents an amino group of the following formula:
- 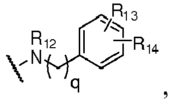 wherein q, R₁₂, R₁₃ and R₁₄ are as defined above, in particular q is 2 or 3, R₁₂ is a hydrogen atom or a (C₁-C₄)alkyl group (such as methyl), R₁₃ is a hydrogen atom, a halogen atom or a (C₁-C₄)alkoxy group (such as methoxy) and R₁₄ is a hydrogen atom.

The present invention comprises the compounds of formula (I) as described above and in particular the combinations of the variants indicated above.

The compounds according to the invention are in particular selected from:
1. (2S)-5-(benzylamino)-N-(2-phenylethyl)-2-[(2-phenylphenyl)formamido]pentanamide
2. (2R)-5-(benzylamino)-N-(2-phenylethyl)-2-[(2-phenylphenyl)formamido]pentanamide
3. (2S)-N-(2-phenylethyl)-5-[(2-phenylethyl)amino]-2-[(2-phenylphenyl)formamido]pentanamide
4. (2S)-5-[(2-methylpropyl)amino]-N-(2-phenylethyl)-2-[(2-phenylphenyl)formamido]pentanamide
5. (2S)-5-(benzylamino)-N-methyl-N-(2-phenylethyl)-2-[(2-phenylphenyl)formamido]pentanamide
6. (2S)-6-(benzylamino)-N-(2-phenylethyl)-2-[(2-phenylphenyl)formamido]hexanamide
7. (2R)-6-(benzylamino)-N-(2-phenylethyl)-2-[(2-phenylphenyl)formamido]hexanamide
8. (2S)-5-{[(2-methoxyphenyl)methyl]amino}-N-(2-phenylethyl)-2-[(2-phenylphenyl)formamido]pentanamide
9. (2S)-5-{[(3-methoxyphenyl)methyl]amino}-N-(2-phenylethyl)-2-[(2-phenylphenyl)formamido]pentanamide
10. (2S)-5-{[(4-methoxyphenyl)methyl]amino}-N-(2-phenylethyl)-2-[(2-phenylphenyl)formamido]pentanamide
11. (2S)-5-(cyclohexylamino)-N-(2-phenylethyl)-2-[(2-phenylphenyl)formamido]pentanamide
12. (2S)-5-{[(2-chlorophenyl)methyl]amino}-N-(2-phenylethyl)-2-[(2-phenylphenyl)formamido]pentanamide
13. (2S)-5- {[(3-chlorophenyl)methyl]amino}-N-(2-phenylethyl)-2-[(2-phenylphenyl)formamido]pentanamide
14. (2S)-5-{[(4-chlorophenyl)methyl]amino}-N-(2-phenylethyl)-2-[(2-phenylphenyl)formamido]pentanamide
15. (2S)-5-[benzyl(methyl)amino]-N-(2-phenylethyl)-2-[(2-phenylphenyl)formamido]pentanamide
16. (2S)-5- {[(2-methoxyphenyl)methyl](methyl)amino}-N-(2-phenylethyl)-2-[(2-phenylphenyl)formamido]pentanamide
17. (2S)-5-{[(3,4-dimethoxyphenyl)methyl](methyl)amino}-N-(2-phenylethyl)-2-[(2-phenylphenyl)formamido]pentanamide
18. (2S)-5-{[(3-chlorophenyl)methyl](methyl)amino}-N-(2-phenylethyl)-2-[(2-phenylphenyl)formamido]pentanamide
19. (2S)-5-{[(2-chlorophenyl)methyl](methyl)amino}-N-(2-phenylethyl)-2-[(2-phenylphenyl)formamido]pentanamide
20. (2S)-5-{[(4-chlorophenyl)methyl](methyl)amino}-N-(2-phenylethyl)-2-[(2-phenylphenyl)formamido]pentanamide
21. (2S)-5- {[(4-methoxyphenyl)methyl](methyl)amino}-N-(2-phenylethyl)-2-[(2-phenylphenyl)formamido]pentanamide
22. (2S)-5- {[(3-methoxyphenyl)methyl](methyl)amino}-N-(2-phenylethyl)-2-[(2-phenylphenyl)formamido]pentanamide
23. (2S)-5-[methyl(2-phenylethyl)amino]-N-(2-phenylethyl)-2-[(2-phenylphenyl)formamido]pentanamide
24. (2S)-N-(2-phenylethyl)-2-[(2-phenylphenyl)formamido]-5-(1,2,3,4-tetrahydroisoquinolin-2-yl)pentanamide
25. (2R)-N-(2-phenylethyl)-2-[(2-phenylphenyl)formamido]-5-(1,2,3,4-tetrahydroisoquinolin-2-yl)pentanamide
26. (2S)-N-(2-phenylethyl)-2-[(2-phenylphenyl)formamido]-5-(piperidin-1-yl)pentanamide
27. (2R)-N-(2-phenylethyl)-2-[(2-phenylphenyl)formamido]-5-(piperidin-1-yl)pentanamide
28. (2S)-2-[(2-phenylphenyl)formamido]-N-(3-phenylpropyl)-5-(piperidin-1-yl)pentanamide
29. (2S)-2-{[2-(4-methoxyphenyl)phenyl]formamido}-N-(3-phenylpropyl)-5-(piperidin-1-yl)pentanamide
30. (2S)-5-(azepan-1-yl)-2-{[2-(4-methoxyphenyl)phenyl]formamido}-N-(3-phenylpropyl)pentanamide
31. (2S)-N-(2-phenylethyl)-2-[(2-phenylphenyl)formamido]-5-(4-phenylpiperidin-1-yl)pentanamide
32. (2S)-5-(4-benzylpiperidin-1-yl)-N-(2-phenylethyl)-2-[(2-phenylphenyl)formamido]pentanamide
33. (2S)-N-(2-phenylethyl)-2-[(2-phenylphenyl)formamido]-5-[4-(piperidin-1-yl)piperidin-1-yl]pentanamide
34. (2S)-5-(4-methylpiperazin-1-yl)-N-(2-phenylethyl)-2-[(2-phenylphenyl)formamido]pentanamide
35. (2S)-5-(4-acetylpiperazin-1-yl)-N-(2-phenylethyl)-2-[(2-phenylphenyl)formamido]pentanamide
36. (2S)-5-(morpholin-4-yl)-N-(2-phenylethyl)-2-[(2-phenylphenyl)formamido]pentanamide
37. (2S)-5-[4-(2-oxo-2,3-dihydro-1H-indol-1-yl)piperidin-1-yl]-N-(2-phenylethyl)-2-[(2-phenylphenyl)formamido]pentanamide
38. (2S)-5-[(1-benzylpiperidin-4-yl)(methyl)amino]-N-(2-phenylethyl)-2-[(2-phenylphenyl)formamido]pentanamide
39. (2S)-5-(4-benzoylpiperazin-1-yl)-N-(2-phenylethyl)-2-[(2-phenylphenyl)formamido]pentanamide
40. (2S)-5-(4-benzylpiperidin-1-yl)-N-(2-phenylethyl)-2-(phenylformamido)pentanamide
41. (2S)-5-(4-benzylpiperidin-1-yl)-2-[(2-chlorophenyl)formamido]-N-(2-phenylethyl)pentanamide
42. (2S)-5-(4-benzylpiperidin-1-yl)-N-(2-phenylethyl)-2-(quinolin-2-ylformamido)pentanamide
43. (2S)-5-(4-benzylpiperidin-1-yl)-2-[(3-chlorophenyl)formamido]-N-(2-phenylethyl)pentanamide
44. (2S)-2-(adamantan-1-ylformamido)-5-(4-benzylpiperidin-1-yl)-N-(2-phenylethyl)pentanamide
45. (2S)-5-(4-benzylpiperidin-1-yl)-2-(3,3-diphenylprop-2-enamido)-N-(2-phenylethyl)pentanamide
46. (2S)-5-(4-benzylpiperidin-1-yl)-N-(2-phenylethyl)-2-[4-(phenylformamido)butanamido]pentanamide; trifluoroacetic acid
47. N-(2-phenylethyl)-2-(phenylformamido)-5-(piperidin-1-yl)pentanamide
48. (2S)-N-[2-(1H-indol-3-yl)ethyl]-2-[(2-phenylphenyl)formamido]-5-(piperidin-1-yl)pentanamide
49. N-[(2S)-1-oxo-5-(piperidin-1-yl)-1-(1,2,3,4-tetrahydroisoquinolin-2-yl)pentan-2-yl]-2-phenylbenzamide
50. (2S)-N-[3-(4-chlorophenyl)propyl]-2-[(2-phenylphenyl)formamido]-5-(piperidin-1-yl)pentanamide
51. (2S)-N-[2-(3,4-dichlorophenyl)ethyl]-2-[(2-phenylphenyl)formamido]-5-(piperidin-1-yl)pentanamide
52. N-[(2S)-1-oxo-5-(piperidin-1-yl)-1-(2,3,4,5-tetrahydro-1H-2-benzazepin-2-yl)pentan-2-yl]-2-phenylbenzamide
53. (2S)-2-[(2-phenylphenyl)formamido]-5-(piperidin-1-yl)-N-{2-[4-(trifluoromethyl)phenyl]ethyl}pentanamide
54. (2S)-2-[(2-phenylphenyl)formamido]-N-(2-phenylpropyl)-5-(piperidin-1-yl)pentanamide
55. (2S)-2-(1-benzofuran-2-ylformamido)-N-(2-phenylethyl)-5-(piperidin-1-yl)pentanamide
56. (2S)-2-(1H-indol-2-ylformamido)-N-(2-phenylethyl)-5-(piperidin-1-yl)pentanamide
57. (2S)-N-(2-phenylethyl)-2-[(2E)-3-phenylprop-2-enamido]-5-(piperidin-1-yl)pentanamide
58. (2S)-2-[3-(1H-indol-3-yl)propanamido]-N-(2-phenylethyl)-5-(piperidin-1-yl)pentanamide
59. (2S)-2-[3-(10H-phenothiazin-10-yl)propanamido]-N-(2-phenylethyl)-5-(piperidin-1-yl)pentanamide
60. (2S)-N-(2-phenylethyl)-5-(piperidin-1-yl)-2-{9H-pyrido[3,4-b]indol-3-ylformamido}pentanamide
61. (2S)-2- {imidazo[1,2-a]pyridin-2-ylformamido} -N-(2-phenylethyl)-5-(piperidin-1-yl)pentanamide
62. N-[(2S)-1-oxo-5-(piperidin-1-yl)-1-(1,2,3,4-tetrahydroisoquinolin-2-yl)pentan-2-yl]-1-benzofuran-2-carboxamide
63. (2S)-2-(1-benzofuran-2-ylformamido)-N-[3-(4-chlorophenyl)propyl]-5-(piperidin-1-yl)pentanamide
64. (2S)-2-(1-benzofuran-2-ylformamido)-N-[(2R)-1-phenylpropan-2-yl]-5-(piperidin-1-yl)pentanamide
65. N-[(2S)-1-oxo-5-(piperidin-1-yl)-1-(1,2,3,4-tetrahydroisoquinolin-2-yl)pentan-2-yl]-3-(10H-phenothiazin-10-yl)propanamide
66. (2S)-N-[3-(4-chlorophenyl)propyl]-2-[3-(10H-phenothiazin-10-yl)propanamido]-5-(piperidin-1-yl)pentanamide
67. (2S)-2-[3-(10H-phenothiazin-10-yl)propanamido]-N-[(2R)-1-phenylpropan-2-yl]-5-(piperidin-1-yl)pentanamide
68. (2E)-N-[(2S)-1-oxo-5-(piperidin-1-yl)-1-(1,2,3,4-tetrahydroisoquinolin-2-yl)pentan-2-yl]-3-phenylprop-2-enamide
69. (2S)-N-[3-(4-chlorophenyl)propyl]-2-[(2E)-3-phenylprop-2-enamido]-5-(piperidin-1-yl)pentanamide
70. (2S)-2-[(2E)-3-phenylprop-2-enamido]-N-[(2R)-1-phenylpropan-2-yl]-5-(piperidin-1-yl)pentanamide

The compounds of formula (I) may be prepared according to techniques known to the person skilled in the art. The present invention describes in this respect various routes of synthesis, which are illustrated in the examples below and may be implemented by the person skilled in the art. The starting compounds may be obtained commercially or may be synthesized according to standard methods. It is understood that the present invention is not limited to a particular route of synthesis, and extends to other methods that enable the production of the indicated compounds. The compounds of the invention can be produced by any chemical or genetic technique commonly known in the art. They can be produced more particularly using a peptide synthesizer with standard solid-phase synthesis approaches (Fmoc/t-butyl or Boc/benzyl chemistry, as described in Merrifield R.B., Science 232, 341-347, 1986). More specifically, compounds of the invention may be prepared by one of the methods described by the following schemes.

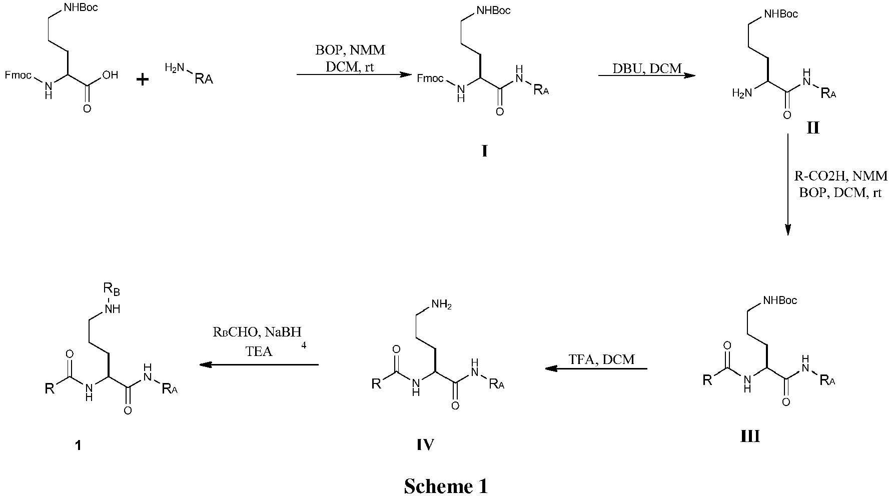

Fmoc-Orn(Boc)-OH was coupled to R_{A}-NH2 in presence of BOP (Benzotriazole-1-yl-oxy-tris-(dimethylamino)-phosphonium hexafluorophosphate), N-methylmorpholine (NMM) in DCM (dichloromethane) at room temperature. Then Fmoc-deprotection was performed with DBU (1,8-diazabicyclo(5.4.0)undec-7-ene), leading to the free amine which was coupled to R-COOH in presence of BOP, NMM in DCM at room temperature. Then acidic hydrolysis by trifluoroacetic acid in dichloromethane led to the corresponding ornithine derivative. Then imine was performed with various R_{B}-CHO in a mixture DCM / Methanol at room temperature in presence of triethylamine (TEA). After 30 minutes, the mixture was cooled down at 0°C, and reduction was performed with sodium borohydride. Of note, -NHR_{B} corresponds to R₁ of formula (I) and -NHR_{A} to R₂ of formula (I).

Fmoc stands for Nα-fluoren-9-ylmethyloxycarbonyl

Boc stands for tert-butyloxycarbonyl

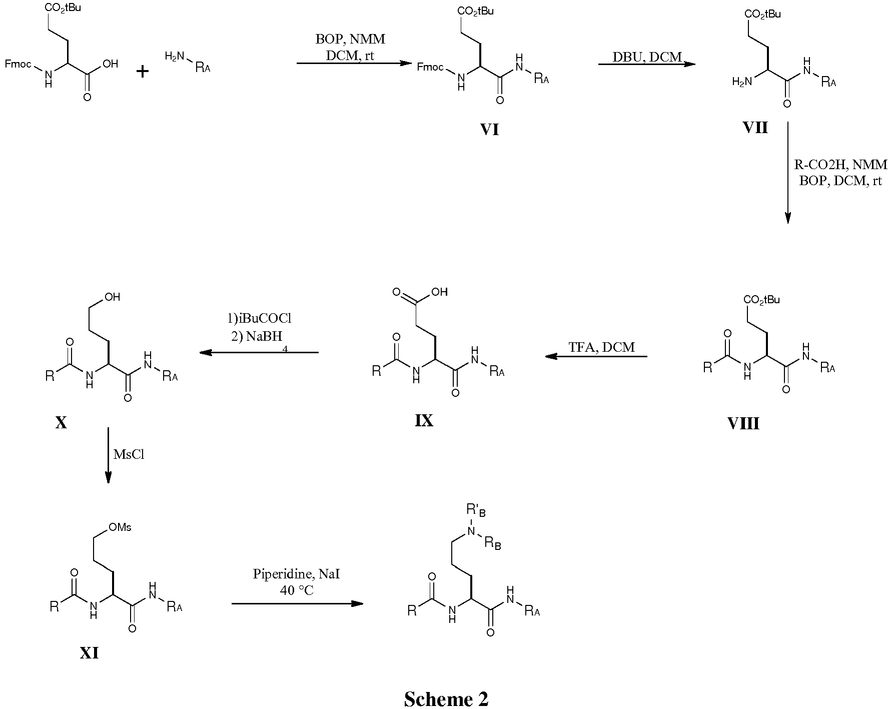

Fmoc-Glu(OtBu)-OH was coupled to R2-NH2 in presence of BOP, N-methylmorpholine (NMM) in DCM at room temperature. Then Fmoc-deprotection was performed with DBU, leading to the free amine which was coupled to R-COOH in presence of BOP, N-methylmorpholine in DCM at room temperature. Then acidic hydrolysis by trifluoroacetic acid in dichloromethane led to the corresponding glutamic acid derivative. Reaction of the carboxylic acid with isobutylchloroformate in presence of NMM in THF at -20°C led to an asymmetric anhydride, which was reduced in situ by addition of sodium borohydride with methanol. The resulting alcohol was activated at 0°C with methanesulfonylchloride in presence of triethylamine in dichloromethane. Substitution of the mesyl group was performed by various amino group (here piperidine group) in dry acetonitrile at 40°C in presence of a catalytic amount of NaI. Of note, - N(R_{B}R'_{B}) corresponds to R₁ of formula (I) and -NHR_{A} to R₂ of formula (I).

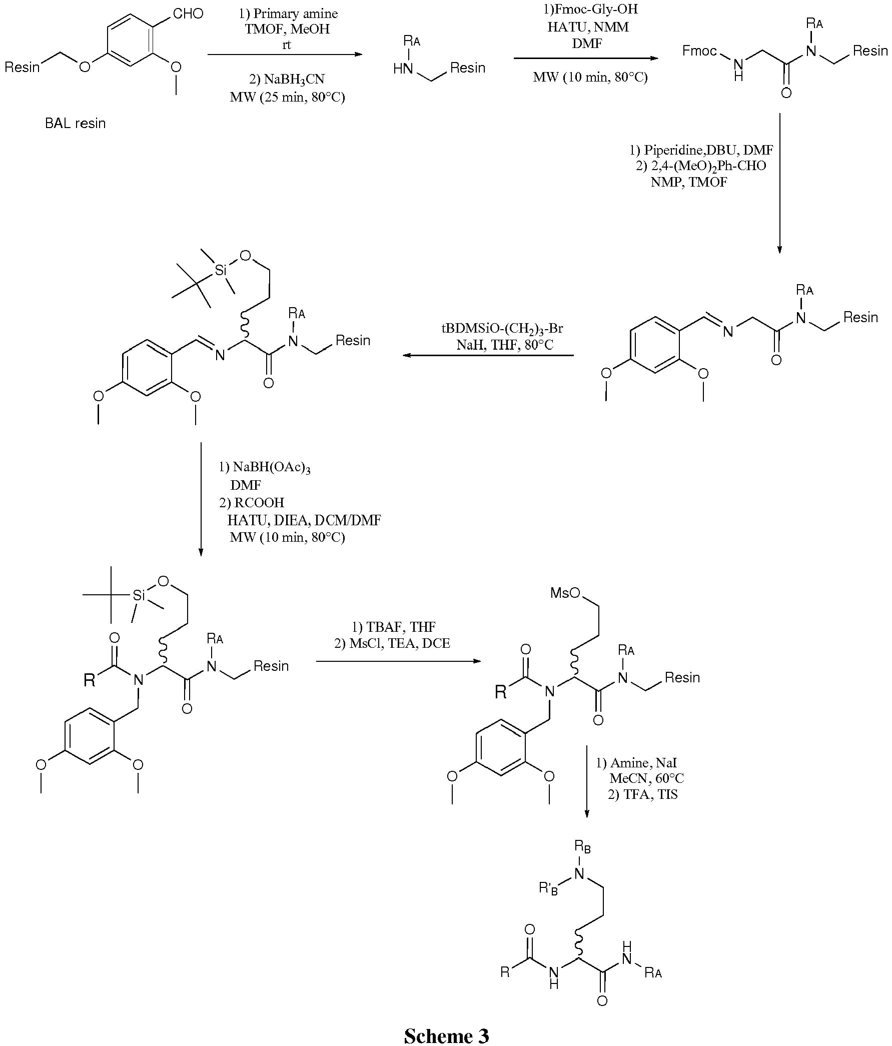

To a suspension of BAL resin in a mixture TMOF/Methanol (2.5:1) was added primary amine (NH₂R_{A}) (10 eq.), and the reaction mixture was stirred at room temperature for 90 minutes. Then, sodium cyanotrihydroborate (10eq.) was added and the reaction mixture was irradiated by microwave at 80°C for 25 minutes and the mixture was stirred at room temperature overnight. After filtration and washes, a mixture of Fmoc-Gly-OH (5 eq.), HATU (4.8 eq.) and DIEA (10 eq.) in DMF/DCM [1:4] was added, and the mixture was irradiated by microwave at 80°C for 10 min. After filtration, the coupling reaction was repeated once. After filtration and washes, resin was treated twice with a solution of piperidine (20% v/v in DMF), for 1 and 20 minutes respectively. After filtration and washes, resin was treated with a solution of 2,4-dimethoxybenzaldehyde (10 eq.) in N-Methylpyrrolidone /TMOF [4:1] overnight. Resin was filtrated and washed. To a suspension of resin in THF were added (3-Bromopropoxy)tertButyldimethylsilane (10 eq.), sodium hydride or potassium tertbutylate (10 eq.), and the reaction mixture was stirred at 80°C for 6 hours. Resin was then filtrated and washed. To a suspension of resin in DMF was added sodium triacetoxyhydroborate (10 eq.), and the reaction mixture was stirred at room temperature overnight. After filtration and washes, resin was treated either with an acyl chloride (10 eq.) in presence of DIEA (20 eq.) for 2 hours at room temperature, or with a carboxylic acid (5 eq.) in presence of HATU (4.9 eq.), DIEA (10 eq.) in DMF/DCM [1:4] and irradiated by microwave at 80°C for 25 min. After filtration, the coupling reaction was repeated once. After filtration and washes, resin was treated with a solution of TBAF (20 eq.) in THF overnight at room temperature. After filtration and washes, resin was then treated with a solution of mesyl chloride (10 eq.) and TEA (10 eq.) in DCM for 7 hours at room temperature. After filtration and washes, resin was treated with amine (20 eq.) in MeCN overnight at 60°C in presence of a catalytic amount of sodium iodide. Cleavage of the resin was performed in a mixture TFA/TIS (97:3) at room temperature for 45 min. Of note, -N(R_{B}R'_{B}) corresponds to R₁ of formula (I) and -NHR_{A} to R₂ of formula (I).

BAL resin stands for 5-(4-Formyl-3,5-dimethoxyphenoxy)pentanoyl aminomethyl polystyrene

TMOF stands for trimethoxyorthoformate

HATU stands for (2-(7-Aza-1H-benzotriazole-1-yl)-1,1,3,3-tetramethyluronium hexafluorophosphate)

DIEA stands for diisopropylethylamine

TBAF stands for tetrabutylammonium fluoride

TIS stands for triisopropylsilane

The compounds according to the invention are powerful NPFF1 and/or NPFF2 receptor ligands (table 1). Ligands are compounds that bind to one or more binding sites of NPFF1 and/or NPFF2 receptors. They can be antagonists or agonists, partially or totally, of NPFF1 or NPFF2 receptors or both.

Certain compounds of the invention have *K*ᵢ<100 nM. Certain compounds show a certain selectivity for NPFF1 or NPFF2 *(e.g.:* compound 26: *K*ᵢ NPFFI=60 nM, *K*ᵢ NPFF2>13 µM; compound 32: *K*ᵢ NPFFI=400 nM, *K*ᵢ NPFF2=34 nM; table 1). In addition to these pharmacological properties, the compounds according to the invention have highly satisfactory *in vivo* activities; they decrease, even block, hyperalgesia induced by administration of opiate analgesics or decrease, even block, exaggerated hypersensitivity to a future nociceptive stimulus.

One object of the invention thus relates to the compounds of general formula (I) according to the invention, including variants, combinations of variants and the specific compounds specified above, as drugs, and to methods for preparing same.

The invention also relates to pharmaceutical compositions comprising the compounds according to the invention and a pharmaceutically acceptable carrier. "Pharmaceutically acceptable carrier" refers to any carrier that is physiologically acceptable to the subject, in particular a human or animal subject, wherein said carrier depends on the type of administration. Of course, said carrier must not block the therapeutic effect of the active compounds present in the composition.

The compounds and the pharmaceutical compositions according to the invention are particularly useful for a therapeutic method and in particular to the treatment of pain. In particular, the compounds and compositions according to the invention decrease or block hyperalgesia and/or tolerance effects related to the use of analgesic compounds, in particular opiate analgesic compounds, or to decrease or block exaggerated hypersensitivity to future nociceptive stimuli.

Thus, the compounds and the pharmaceutical compositions according to the invention may be used in the treatment of postoperative pain or of severe chronic pain caused by inflammation, neuropathy, cancer, diabetes or drugs.

The invention also relates to a method for treating pain in a subject, comprising the administration to said subject of an effective quantity of the compound or the pharmaceutical composition according to the invention.

The invention also relates to the use of at least one compound according to the invention for preparing a pharmaceutical composition intended to treat pain, in particular to decrease or block hypersensitivity to pain or to decrease or block hyperalgesia and/or tolerance effects related to the use of analgesic compounds, in particular opiate analgesic compounds.

In the case where the compound or the pharmaceutical composition according to the invention is intended to decrease or block hyperalgesia and/or tolerance effects induced by the use of an analgesic compound, in particular an opiate analgesic compound, the compound or the pharmaceutical composition containing said compound may be administered simultaneously with, separately from or sequentially to the analgesic compound.

According to a particular variant, one object of the invention relates to a pharmaceutical composition comprising at least one compound according to the invention, at least one analgesic compound, in particular an opiate, and a pharmaceutically acceptable carrier.

### The analgesic compounds

The analgesic compounds used in the context of the present invention are generally opiate compounds, i.e., compounds that act on opioid receptors. They are generally used to treat severe and long-lasting pain. Preferably, these are morphine compounds, notably morphine or morphinomimetic compounds, i.e., compounds that are derived from morphine and/or that act on morphine receptors and/or that recruit one or more metabolic pathways common to morphine. As particular examples, mention may be made of the following compounds in particular: morphine, fentanyl, sufentanil, alfentanyl, heroin, oxycodone, hydromorphone, levorphanol, methadone, buprenorphine, butorphanol, meperidine, etc.

The invention is quite particularly suited to the inhibition of hyperalgesia induced by morphine, fentanyl or heroin.

The term "treatment" comprises a curative treatment as well as a prophylactic treatment of pain. A curative treatment is defined as a treatment that eases, improves and/or eliminates, reduces and/or stabilizes suffering or pain. A prophylactic treatment comprises a treatment that prevents pain as well as a treatment that reduces and/or delays pain or the risk of the occurrence of pain.

By decreasing or blocking hyperalgesia and/or tolerance effects related to the use of opiates, the compounds according to the invention prolong the duration of action of opiates and/or increase the intensity of their analgesic effect, without causing hypersensitivity to pain. The growing need to increase the doses of opiates in order to maintain the same analgesic effect is thus decreased, even absent.

Generally, it appears that the administration of opiate analgesics to mammalian subjects is always accompanied by hyperalgesia, and thus the compound according to the invention may be used each time an opiate analgesic is administered to a subject.

Furthermore, as specified above, the administration of high doses of opiates leads to a certain number of side effects such as nausea, constipation, sedation and respiratory deficiencies *(e.g.:* delayed respiratory depression). The compounds according to the invention allows to use lower doses of opiates and should therefore limit adverse side effects of opiates, such as nausea, constipation, sedation or respiratory deficiencies, including delayed respiratory depression.

Furthermore, the effect of the compounds according to the invention on hypersensitivity to pain induced by opiates makes it possible to also envisage the administration of said compounds alone in the context of the prophylactic treatment of pain.

Hyperalgesia induced by stress or by opioids may be prolonged or brief, significant or moderate. The detection, measurement and characterization of the presence of hyperalgesia may be carried out by standard clinical tests (observation, etc.).

In the context of the present invention, the term "inhibit" means to decrease or block (or reduce or suppress) in a partial or total, transitory or prolonged manner. Thus, such terms in the present description are used interchangeably. The capacity to inhibit hyperalgesia and the degree of such inhibition may be determined according to various tests known to the person skilled in the art. Furthermore, the term "inhibit" refers to inhibition of the appearance of hyperalgesia (for a preventive treatment, for example) as well as to inhibition of the development or duration of hyperalgesia (for a curative treatment).

The compounds or compositions according to the invention may be administered in various ways and in various forms. Thus, they may be injected by oral or more generally by a systemic route, such as, for example, by intravenous, intramuscular, subcutaneous, transdermal, intra-arterial route, etc. Preferably, the compounds or compositions according to the invention are administered by oral route. For injections, the compounds are generally packaged in the form of liquid suspensions, which may be injected via syringes or perfusions, for example. In this respect, the compounds are generally dissolved in saline, physiological, isotonic or buffered solutions, etc., compatible with pharmaceutical use and known to the person skilled in the art. Thus, the compositions may contain one or more agents or excipients selected from dispersants, solubilizers, stabilizers, preservatives, etc. Agents or excipients that can be used in liquid and/or injectable formulations are notably methylcellulose, hydroxymethylcellulose, carboxymethylcellulose, polysorbate 80, mannitol, gelatin, lactose, vegetable oils, acacia, etc.

According to a particular variant, the compound according to the invention is administered by the same route as the analgesic compound, for example by oral route.

The compounds may also be administered in the form of gels, oils, tablets, suppositories, powders, gelatin capsules, capsules, etc., optionally by means of dosage forms or devices that ensure prolonged and/or delayed release. For this type of formulation, an agent such as cellulose, carbonate or starch is advantageously used.

It is understood that the flow rate and/or dose administered may be adjusted by the person skilled in the art according to the patient, the pain observed, the analgesic concerned, the mode of administration, etc. Typically, the compounds are administered at doses that may vary between 0.1 µg and 10 mg/kg of body weight, more generally from 1 µg to 1000 µg/kg. Furthermore, administration by oral route or by injection may comprise several (2, 3 or 4) administrations per day, if need be.

In addition, for chronic treatments, delayed or prolonged systems may be advantageous, ensuring the subject effective and long-lasting pain treatment.

The present invention may be used for the preventive or curative treatment of hyperalgesia in multiple situations, such as that occurring or associated with acute or chronic pain in response to surgery, trauma or pathology of a mammal.

It may be applied to any mammal, in particular humans, but also to animals, in particular domestic or breeding animals, in particular horses, dogs, etc.

It is particularly suited for preventing or treating sensitization processes induced by the limited administration of opiate analgesics, such as powerful morphinomimetics (morphine or fentanyl or derivatives thereof, for example), during surgical or trauma procedures.

It may also be used to prevent or treat chronic pain in mammals (particularly patients) suffering from pathologies such as cancer, bums, etc., for which generally analgesics (such as morphine) may be administered for a long period, optionally in delayed form.

The compounds according to the invention may also be used to prevent or reduce, in a highly significant manner, tolerance processes, thus making it possible to reduce daily doses of morphine and thus to improve the clinical picture of patients (side effects of morphinomimetics, such as intestinal disorders, for example).

Reversal of opiate-induced hyperalgesia makes it possible to maintain opiate effectiveness over time and thus to use lower doses of analgesics, which in turn causes fewer side effects.

The compounds of formula (I) according to the invention may also be used for the preventive or curative treatment of pain.

The compounds of formula (I) according to the invention may also be used for the treatment of opiate dependence (drug addiction).

According to a specific embodiment, the invention also relates to a kit that is suitable for the treatment by the methods described above. These kits comprise a composition containing the compound of the invention in the dosages indicated above and a second composition containing an analgesic compound, preferably an opiate compound, in the dosages indicated above, for a simultaneous, separate or sequential administration, in effective amounts according to the invention.

Other aspects and advantages of the present invention will become apparent upon consideration of the following examples, which must be regarded as illustrative and nonrestrictive.

### EXAMPLES

### Example 1 : Synthesis of compound 1

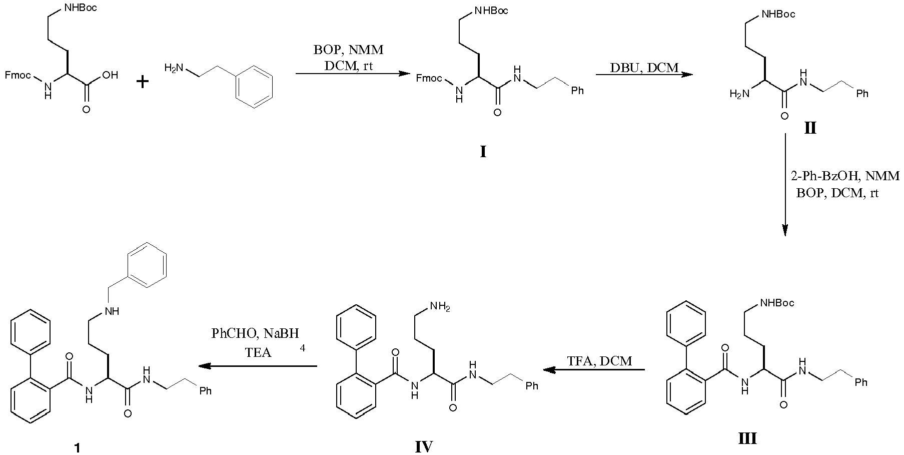

### (2S)-5-(benzylamino)-N-(2-phenylethyl)-2-[(2-phenylphenyl)formamido]pentanamide (1)

To a solution of Fmoc-Orn(Boc)-OH (1 eq., 4 g) in DCM (40 mL) were added BOP (1.1 eq., 4.28 g) and NMM (1.2 eq., 713 µL) and the mixture was stirred at room temperature for 10 minutes, before adding phenethylamine (1.2 eq., 1.06 mL). The reaction mixture was stirred under argon at room temperature (rt) overnight. The resulting slurry was filtered and dried under vacuum. The resulting white powder was solubilized in EtOAc, and successively washed with aqueous hydrochloric acid (1N), saturated aqueous sodium bicarbonate and brine, then dried over sodium sulfate. The solution was filtered and concentrated under vacuum. No further purification step was performed and compound **I** was obtained as a white powder in 79% yield (3.87 g). ¹H NMR (400 MHz, DMSO-d⁶) δ 7.93 (br. s, 1H), 7.92 (d, *J =* 7.2 Hz, 2H), 7.74 (d, *J =* 7.2 Hz, 2H), 7.42 (dd, *J =* 7.6 Hz, 2H), 7.33 (dd, *J =* 7.2 Hz, 2H), 7.25 - 7.30 (m, 2H), 7.15 - 7.25 (m, 3H), 6.78 (br. s, 1H), 4.18 - 4.26 (m, 3H), 3.88 - 3.98 (m, 1H), 3.20 - 3.40 (m, 2H), 2.85 - 2.98 (m, 2H), 2.71 (t, J = 7.6 Hz, 2H), 1.40 - 1.62 (m, 4H), 1.38 (s, 9H). To a suspension of **I** (1 eq, 2.97 g) in DMF (30 mL) was added piperidine (6 mL), and the reaction mixture was stirred at room temperature overnight. The solution was concentrated under vacuum, and immediately purified by chromatography on silica gel (eluent MeOH 2% to 20% in DCM), affording the corresponding compound **II** in 98% yield (2.28 g). ¹H NMR (400 MHz, CDCl₃) δ 7.93 (s, 1H), 7.20 - 7.30 (m, 3H), 7.10-7.20 (m, 3H), 3.71 (br. s, 1 H), 3.39 - 3.51 (m, 2H), 2.95 - 3.10 (m, 2H), 2.75 (t, *J =* 7.2 Hz, 2H), 1.62 - 1.75 (m, 1H), 1.39 - 1.49 (m, 3H), 1.38 (s, 9H).

Due to the carbonatation side-reaction of the free-amino function, compound **II** (1 eq., 2.24 g) was quickly added to a solution of o-Phenylbenzoic acid (1.5 eq., 1.99 g), BOP (1.5 eq., 4.43 g) and NMM (1.5 eq., 1.01 mL) in DCM (30 mL). The reaction mixture was stirred at room temperature overnight. After evaporation under vacuum, the resulting slurry was solubilized in EtOAc, and successively washed with saturated aqueous sodium bicarbonate and brine, then dried over sodium sulfate. The solution was filtered and concentrated under vacuum. The resulting slurry was precipitated in mixture DCM/Diethyl ether to afford **III** as a white powder in 83% yield (2.86 g). To a solution of **III** (2.86 g) in DCM (25 mL) at 0°C was slowly added TFA (25 mL), and the reaction mixture was stirred at room temperature for 1 hour. After evaporation under vacuum, the resulting TFA salt of **IV** was obtained as a white powder in quantitative yield (2.9 g). ¹H NMR (400 MHz, CDCl₃) δ 7.51 (s, 1H), 7.35 - 7.45 (m, 2H), 7.00 - 7.35 (m, 15H), 6.66 (d, *J* = 7.2 Hz, 1H), 4.31 (br. s, 1H), 3.15 - 3.30 (m, 2H), 2.70 - 2.88 (m, 2H), 2.52 - 2.68 (m, 2H), 1.48 - 1.62 (m, 1H), 1.25 - 1.46 (m, 3H). LRMS (ESI) (C₂₆H₃₀N₃O₂) [M + H]⁺ 416.2.

To a solution of **IV** (1 eq., 32 mg) in dry DCM (0.5 mL) under argon, were added TEA (4eq., 34 µl) and a solution of benzaldehyde (2 eq., 12 µL) in 0.5 mL of dry methanol. After 30 minutes of stirring at room temperature, the reaction mixture was cooled down at 0°C, and sodium borohydrure (3eq., 7 mg) was added. The reaction mixture was stirred at room temperature for 1 hour. Few drops of water were added to the reaction solution, and volatiles were removed in vacuo. The resulting slurry was solubilized in EtOAc, and successively washed with saturated aqueous sodium bicarbonate and brine, then dried over sodium sulfate. The solution was filtered and concentrated under vacuum. Purification was performed by reverse phase LPLC (20% to 100% MeOH in water with 0.05% TFA, 25 min) affording 1 (13 mg) in 43% yield. ¹H NMR (400 MHz, MeOD) δ 7.34 - 7.58 (m, 13H), 7.28 - 7.33 (m, 2H), 7.20 - 7.26 (m, 4H), 4.34 (dd, *J* = 5.50, 7.70 Hz, 1H), 4.19 (s, 2H), 3.38 (t, *J* = 7.40 Hz, 2H), 2.97 (t, *J* = 7.30 Hz, 2H), 2.78 (t, *J* = 7.50 Hz, 2H), 1.65 - 1.78 (m, 1H), 1.46 - 1.65 (m, 3H). LRMS (ESI) (C₃₃H₃₅N₃O₂) [M + H]⁺ 506.2.

Compounds **2** to **14** were prepared by analogous variation of these synthetic procedures:
● (2R)-5-(benzylamino)-N-(2-phenylethyl)-2-[(2-phenylphenyl)formamido]pentanamide **(2)** ¹H NMR (400 MHz, MeOD) δ 7.33 - 7.56 (m, 13H), 7.16 - 7.32 (m, 6H), 4.31 (dd, J = 5.50, 7.50 Hz, 1H), 4.16 (s, 2H), 3.35 (t, *J* = 7.30 Hz, 2H), 2.94 (t, *J* = 7.53 Hz, 2H), 2.75 (t, *J* = 7.30 Hz, 2H), 1.62 - 1.76 (m., 1H), 1.41 - 1.62 (m, 3H). LRMS (ESI) (C₃₃H₃₅N₃O₂) [M + H]⁺ 506.2.
● (2S)-N-(2-phenylethyl)-5-[(2-phenylethyl)amino]-2-[(2-phenylphenyl)formamido]pentanamide **(3)** LRMS (ESI) (C₃₄H₃₇N₃O₂) [M + H]⁺ 520.2.
● (2S)-5-[(2-methylpropyl)amino]-N-(2-phenylethyl)-2-[(2-phenylphenyl)formamido]pentanamide **(4)** LRMS (ESI) (C₃₀H₃₇N₃O₂) [M + H]⁺ 472.1.
● (2S)-5-(benzylamino)-N-methyl-N-(2-phenylethyl)-2-[(2-phenylphenyl)formamido]pentanamide (5) ¹H NMR (400 MHz, MeOD) δ 7.15 - 7.60 (m, 38H), 4.72 - 4.80 (m, 1H), 4.51 - 4.59 (m, 1H), 4.15 (s, 2H), 4.17 (s, 2H), 3.68 - 3.80 (m, 2H), 3.45 - 3.60 (m, 2H), 2.88 (s, 3H), 2.94 (s, 3H), 2.82 - 3.00 (m, 4H), 2.79 (t, *J =* 7.30 Hz, 4H), 1.25 - 1.69 (m, 8H). LRMS (ESI) (C₃₄H₃₇N₃O₂) [M + H]⁺ 520.2.
● (2S)-6-(benzylamino)-N-(2-phenylethyl)-2-[(2-phenylphenyl)formamido]hexanamide **(6)** LRMS (ESI) (C₃₄H₃₇N₃O₂) [M + H]⁺ 520.2.
● (2R)-6-(benzylamino)-N-(2-phenylethyl)-2-[(2-phenylphenyl)formamido]hexanamide **(7)** LRMS (ESI) (C₃₄H₃₇N₃O₂) [M + H]⁺ 520.2.
● (2S)-5-{[(2-methoxyphenyl)methyl]amino}-N-(2-phenylethyl)-2-[(2-phenylphenyl)formamido]pentanamide (8) ¹H NMR (400 MHz, MeOD) δ 7.23 - 7.60 (m, 12H), 7.21 (m, 4H), 7.12 (d, *J=* 8.53 Hz, 1H), 7.03 (dt, *J* = 1.00, 7.53 Hz, 1H), 4.30 (dd, *J* = 5.40, 7.50 Hz, 1H), 4.15 (s, 2H), 3.93 (s, 3H), 3.35 (t, *J* = 7.40 Hz, 2H), 2.92 (t, *J* = 7.30 Hz, 2H), 2.75 (t, *J* = 7.40 Hz, 2H), 1.60 - 1.74 (m, 1H), 1.39 - 1.60 (m, 3H). LRMS (ESI) (C₃₄H₃₇N₃O₃) [M + H]⁺ 536.2.
● (2S)-5-{[(3-methoxyphenyl)methyl]amino}-N-(2-phenylethyl)-2-[(2-phenylphenyl)formamido]pentanamide **(9)** LRMS (ESI) (C₃₄H₃₇N₃O₃) [M + H]⁺ 536.2.
● (2S)-5-{[(4-methoxyphenyl)methyl]amino}-N-(2-phenylethyl)-2-[(2-phenylphenyl)formamido]pentanamide (10) ¹H NMR (400 MHz, MeOD) δ 7.24 - 7.60 (m, 13H), 7.16 - 7.23 (m, 3H), 7.01 (d, *J* = 8.78 Hz, 2H), 4.26 - 4.34 (m, 1H), 4.09 (s, 2H), 3.82 (s, 3H), 3.34 - 3.37 (m, 2H), 2.86 - 2.96 (m, 2H), 2.74 (t, *J=* 7.50 Hz, 2H), 1.61 - 1.72 (m, 1H), 1.40 - 1.59 (m, 3H). LRMS (ESI) (C₃₄H₃₇N₃O₃) [M + H]⁺ 536.2.
● (2S)-5-(cyclohexylamino)-N-(2-phenylethyl)-2-[(2-phenylphenyl)formamido]pentanamide **(11)** LRMS (ESI) (C₃₂H₃₉N₃O₂) [M + H]⁺ 498.2.
● (2S)-5-{[(2-chlorophenyl)methyl]amino}-N-(2-phenylethyl)-2-[(2-phenylphenyl)formamido]pentanamide **(12)** LRMS (ESI) (C₃₃H₃₄ClN₃O₂) [M + H]⁺ 540.1.
● (2S)-5-{[(3-chlorophenyl)methyl]amino}-N-(2-phenylethyl)-2-[(2-phenylphenyl)formamido]pentanamide **(13)** ¹H NMR (400 MHz, MeOD) δ 7.31 - 7.59 (m, 13H), 7.25 - 7.31 (m, 2H), 7.17 - 7.23 (m, 3H), 4.31 (dd, *J=* 5.40, 7.50 Hz, 1H), 4.16 (s, 2H), 3.34 (t, *J=* 7.53 Hz, 2H), 2.89 - 3.01 (m, 2H), 2.74 (t, *J=* 7.40 Hz, 2H), 1.62 - 1.74 (m, 1H), 1.42 - 1.61 (m, 3H). LRMS (ESI) (C₃₃H₃₄ClN₃O₂) [M + H]⁺ 540.1.
● (2S)-5-{[(4-chlorophenyl)methyl]amino}-N-(2-phenylethyl)-2-[(2-phenylphenyl)formamido]pentanamide **(14)** ¹H NMR (400 MHz, MeOD) δ 7.45 - 7.57 (m, 6H), 7.31 - 7.45 (m, 7H), 7.25 - 7.31 (m, 2H), 7.20 (d, *J=* 6.78 Hz, 3H), 4.31 (dd, *J=* 5.50, 7.40 Hz, 1H), 4.15 (s, 2H), 3.34 (t, *J* = 7.50 Hz, 2H), 2.96 (t, J = 7.28 Hz, 2H), 2.74 (t, J = 7.40 Hz, 2H), 1.62 - 1.75 (m, 1H), 1.41 - 1.62 (m, 3H). LRMS (ESI) (C₃₃H₃₄ClN₃O₂) [M + H]⁺ 540.1.

### Example 2 : Synthesis of compound 26

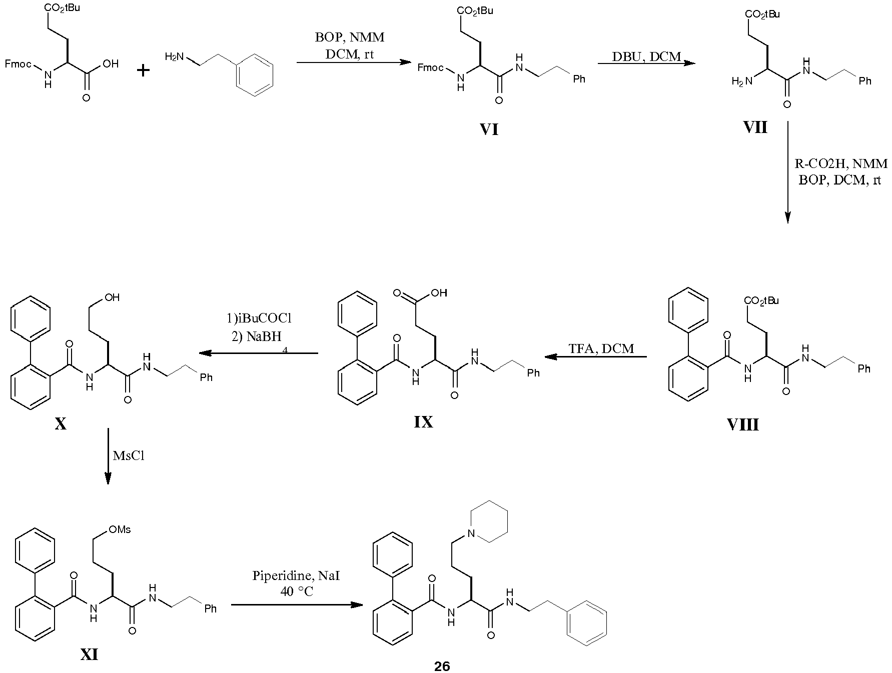

### (2S)-N-(2-phenylethyl)-2-[(2-phenylphenyl)formamido]-5-(piperidin-1-yl)pentanamide (26)

To a solution of Fmoc-Glu(tBu)-OH (1 eq., 2.5 g) in DCM (15 mL) were added BOP (1.2 eq., 3.12 g) and NMM (1.2 eq., 713 µL) and the mixture was stirred at room temperature for 10 minutes, before adding phenethylamine (1.2 eq., 854 µL). The reaction mixture was stirred under argon at rt overnight. After evaporation under vacuum, the resulting slurry was then triturated in ethanol and filtered, affording compound **VI** as a white powder in 96% yield (2.98g). Traces of HMPA were detected by ¹H NMR. ¹H NMR (400 MHz, CDCl₃) δ 7.69 (d, *J* = 7.53 Hz, 2H), 7.51 (d, *J* = 7.28 Hz, 2H), 7.33 (t, *J =* 7.30 Hz, 2H), 7.24 (dt, *J =* 1.26, 7.53 Hz, 2H), 7.17 - 7.21 (m, 2H), 7.04 - 7.15 (m, 3H), 6.25 (br. s, 1H), 5.56 - 5.74 (m, 1H), 4.21 - 4.36 (m, 2H), 4.12 (t, *J* = 7.30 Hz, 1H), 3.99 - 4.09 (m, 1H), 3.46 (br. s., 2H), 2.74 (t, *J =* 7.03 Hz, 2H), 2.22 - 2.39 (m, 1H), 2.06 - 2.22 (m, 1H), 1.87 - 2.05 (m, 1H), 1.73 - 1.87 (m, 1H), 1.37 (s, 9H). LRMS (ESI) (C₃₂H₃₆N₂O₅) [M + H]⁺ 529.2.

To a suspension of **VI** (1 eq, 2.97 g) in DCM (15 mL) was added dropwise DBU (2 eq., 1.69 mL), and the reaction mixture was stirred at room temperature overnight. The solution was concentrated under vacuum, and immediately purified by chromatography on silica gel (eluent MeOH 2% to 20% in DCM), affording the corresponding compound **VII** in 94% yield (1.61 g). ¹H NMR (400 MHz, CDCl₃) δ 7.18 - 7.28 (m, 3H), 7.09 - 7.18 (m, 3H), 3.40 - 3.50 (m, 2H), 3.28 (dd, *J =* 5.27, 7.28 Hz, 1H), 2.76 (t, *J* = 7.03 Hz, 2H), 2.16 - 2.28 (m, 2H), 1.92 - 2.03 (m, 1H), 1.70 (qd, *J =* 7.28, 14.31 Hz, 1H), 1.52 (br. s., 2H), 1.36 (s, 9H)

Due to the carbonatation side-reaction of the free-amino function, compound **VII** (1 eq., 1.17 g) was quickly added to a solution of o-Phenylbenzoic acid (1 eq., 0.76 g), BOP (1.1 eq., 1.9 g) and NMM (1.1 eq., 0.46 mL) in DCM (20 mL). The reaction mixture was stirred at room temperature overnight. After evaporation under vacuum, the resulting slurry was solubilized in EtOAc, and successively washed with aqueous hydrochloric acid (1N), saturated aqueous sodium bicarbonate and brine, then dried over sodium sulfate. The solution was filtered and concentrated under vacuum. Purification was performed by chromatography on silica gel (eluent MeOH 2% in DCM), affording the corresponding compound **VIII** as a white powder in 81% yield (1.5 g). ¹H NMR (300 MHz, CDCl₃) δ 7.61 (d, *J =* 7.49 Hz, 1H), 7.14 - 7.53 (m, 13H), 6.25 (br. s., 1H), 6.17 (d, *J =* 7.80 Hz, 1H), 4.39 (dd, *J =* 0.62, 5.62 Hz, 1H), 3.30 - 3.52 (m, 2H), 2.75 (t, *J=* 7.18 Hz, 2H), 1.89 - 2.20 (m, 2H), 1.72 - 1.87 (m, 1H), 1.51 - 1.66 (m, 1H), 1.43 (s, 9H). LRMS (ESI) (C₃₀H₃₄N₂O₄) [M + Na]⁺ 509.2.

To a solution of **VIII** (1.5 g) in DCM (9 mL) was slowly added TFA (7 mL), and the reaction mixture was stirred at room temperature for 2 hours. After evaporation under vacuum, the resulting slurry was triturated in diethyl ether, affording after filtration compound **IX** as a white powder in quantitative yield (1.3 g). ¹H NMR (300 MHz, CDCl₃) δ 7.05 - 7.68 (m, 14H), 6.72 (br. s, 1H), 6.33 (d, *J =* 8.11 Hz, 1H), 4.41 - 4.58 (m, 1H), 3.29 - 3.54 (m, 2H), 2.74 (t, *J =* 7.18 Hz, 2H), 2.00 - 2.26 (m, 2H), 1.68 - 1.87 (m, 1H), 1.45 - 1.64 (m, 1H)

To a solution of **IX** (1.5 g) in THF at -20°C, were added dropwise isobutylchloroformate (2.1 eq., 950 µL) and NMM (2.1 eq., 800 µL), and the reaction mixture was stirred at -20°C for 3 hours. Then, sodium borohydrure (5 eq., 660 mg) was slowly added to the solution, followed by 36 mL of methanol. The reaction mixture was stirred at -20°C for 35 minutes. After completion, the reaction was quenched by a solution of aqueous hydrochloric acid (20 mL), and concentrated under vacuum. 20 mL of EtOAc were added and successively washed with aqueous hydrochloric acid (1N), saturated aqueous sodium bicarbonate and brine, then dried over sodium sulfate. The solution was filtered and concentrated under vacuum. Purification was performed by chromatography on silica gel (eluent MeOH 2% to 15% in DCM), affording the corresponding alcohol **X** as yellowish oil in 90% yield (1.3 g). ¹H NMR (400 MHz, CDCl₃) δ 7.53 (dd, *J =* 1.63, 7.66 Hz, 1H), 7.38 - 7.45 (m, 1H), 7.25 - 7.36 (m, 6H), 7.17 - 7.24 (m, 3H), 7.11 - 7.17 (m, 1H), 7.06 - 7.11 (m, 2H), 6.18 (t, *J =* 6.53 Hz, 1H), 6.01 (d, *J =* 7.78 Hz, 1H), 4.30 - 4.42 (m, 1H), 3.23 - 3.49 (m, 4H), 2.67 (t, *J =* 7.03 Hz, 2H), 1.44 - 1.57 (m, 1H), 1.30 - 1.42 (m, 1H), 1.11 - 1.24 (m, 2H)

To a solution of **X** (1 eq., 1.25 g) in dry DCM at 0°C were added dropwise TEA (2.5 eq., 1.05 mL) and methanesulfonylchloride (2.5 eq., 582 µL). The reaction was warmed to room temperature, and stirred overnight. After evaporation under vacuum, the resulting slurry was solubilized in EtOAc, and successively washed with brine, then dried over sodium sulfate. The solution was filtered and concentrated under vacuum. Purification was performed by chromatography on silica gel (eluent MeOH 5% in DCM), affording the corresponding compound **XI** in 85% yield (1.25 g). ¹H NMR (400 MHz, CDCl₃) δ 7.53 (dd, *J =* 1.51, 7.53 Hz, 1H), 7.39 - 7.46 (m, 1H), 7.25 - 7.38 (m, 7H), 7.19 - 7.25 (m, 2H), 7.12 - 7.17 (m, 1H), 7.06 - 7.11 (m, 2H), 6.05 (t, *J =* 6.02 Hz, 1H), 5.82 (d, *J =* 8.03 Hz, 1H), 4.28 - 4.37 (m, 1H), 4.07 - 4.16 (m, 1H), 3.97 - 4.05 (m, 1H), 3.23 - 3.44 (m, 2H), 2.91 (s, 3H), 2.67 (t, *J =* 7.28 Hz, 2H), 1.46 - 1.67 (m, 2H), 1.15 - 1.43 (m, 2H)

To a solution of **XI** (1 eq., 1 g) in dry acetonitrile (15 mL) were added sodium iodide (0.1 eq., 30 mg) and piperidine (3.5 eq., 700 µL), and the reaction mixture was stirred at 40°C overnight. After evaporation under vacuum, the resulting slurry was solubilized in EtOAc, and successively washed with saturated aqueous sodium bicarbonate and brine, then dried over sodium sulfate. The solution was filtered and concentrated under vacuum. Purification was performed by chromatography on silica gel (eluent MeOH 2% to 20% in DCM), affording the corresponding **26** as an oil in 74% yield (724 mg). ¹H NMR (400 MHz, CDCl₃) δ 7.72 (t, *J =* 5.77 Hz, 1H), 7.56 (dd, *J =* 1.38, 7.65 Hz, 1H), 7.41 - 7.48 (m, 1H), 7.28 - 7.40 (m, 6H), 7.12 - 7.26 (m, 4H), 6.88 (d, *J=* 7.53 Hz, 1H), 6.48 (br. s., 2H), 4.39 - 4.55 (m, 1H), 3.42 - 3.57 (m, 2H), 3.31 - 3.43 (m, 2H), 3.07 - 3.28 (m, 2H), 2.79 (t, *J =* 7.80 Hz, 2H), 2.47 - 2.64 (m, 2H), 1.84 - 1.99 (m, 2H), 1.63 - 1.83 (m, 5H), 1.43 - 1.63 (m, 2H), 1.21 - 1.40 (m, 1H). LRMS (ESI) (C₃₁H₃₇N₃O₂) [M + H]⁺ 484.2.Treatment of **26** by 1 eq. of sulfuric acid in DCM led after filtration to a sulfate salt of **26** as a white powder.

Compounds **15** to **25** and **27** to **47** were prepared by analogous variation of these synthetic procedures:
● (2S)-5-[benzyl(methyl)amino]-N-(2-phenylethyl)-2-[(2-phenylphenyl)formamido]pentanamide **(15)** ¹H NMR (400 MHz, MeOD) δ 7.14 - 7.57 (m, 19H), 4.11 - 4.41 (m, 3H), 3.33 - 3.40 (m, 2H), 2.91 - 3.18 (m, 2H), 2.76 (t, *J =* 7.40 Hz, 2H), 2.71 (s, 3H), 1.37 - 1.68 (m, 4H) LRMS (ESI) (C₃₄H₃₇N₃O₂) [M + H]⁺ 520.3
● (2S)-5-{[(2-methoxyphenyl)methyl](methyl)amino}-N-(2-phenylethyl)-2-[(2-phenylphenyl)formamido]pentanamide **(16)**
   LRMS (ESI) (C₃₅H₃₉N₃O₃) [M + H]⁺ 550.3.
● (2S)-5-{[(3,4-dimethoxyphenyl)methyl](methyl)amino}-N-(2-phenylethyl)-2-[(2-phenylphenyl)formamido]pentanamide **(17)**
   LRMS (ESI) (C₃₆H₄₁N₃O₄) [M + H]⁺ 580.3.
● (2S)-5-{[(3-chlorophenyl)methyl](methyl)amino}-N-(2-phenylethyl)-2-[(2-phenylphenyl)formamido]pentanamide **(18)**
   LRMS (ESI) (C₃₄H₃₆ClN₃O₂) [M + H]⁺ 554.2.
● (2S)-5-{[(2-chlorophenyl)methyl](methyl)amino}-N-(2-phenylethyl)-2-[(2-phenylphenyl)formamido]pentanamide **(19)**
   LRMS (ESI) (C₃₄H₃₆ClN₃O₂) [M + H]⁺ 554.2.
● (2S)-5-{[(4-chlorophenyl)methyl](methyl)amino}-N-(2-phenylethyl)-2-[(2-phenylphenyl)formamido]pentanamide **(20)**
   LRMS (ESI) (C₃₄H₃₆ClN₃O₂) [M + H]⁺ 554.2.
● (2S)-5-{[(4-methoxyphenyl)methyl](methyl)amino}-N-(2-phenylethyl)-2-[(2-phenylphenyl)formamido]pentanamide **(21)**
   LRMS (ESI) (C₃₅H₃₉N₃O₃) [M + H]⁺ 550.3.
● (2S)-5-{[(3-methoxyphenyl)methyl](methyl)amino}-N-(2-phenylethyl)-2-[(2-phenylphenyl)formamido]pentanamide **(22)**
   LRMS (ESI) (C₃₅H₃₉N₃O₃) [M + H]⁺ 550.3.
● (2S)-5-[methyl(2-phenylethyl)amino]-N-(2-phenylethyl)-2-[(2-phenylphenyl)formamido]pentanamide **(23)**
   ¹H NMR (400 MHz, MeOD) δ 7.47 - 7.55 (m, 2H), 7.23 - 7.46 (m, 12H), 7.12 - 7.22 (m, 6H), 4.26 (dd, *J =* 5.52, 8.28 Hz, 1H), 3.32 - 3.43 (m, 2H), 2.72 - 2.82 (m, 4H), 2.63 - 2.71 (m, 2H), 2.46 (t, *J =* 7.50 Hz, 2H), 2.34 (s, 3H), 1.41 - 1.63 (m, 2H), 1.23 - 1.41 (m, 2H). LRMS (ESI) (C₃₅H₃₉N₃O₂) [M + H]⁺ 534.2.
● (2S)-N-(2-phenylethyl)-2-[(2-phenylphenyl)formamido]-5-(1,2,3,4-tetrahydroisoquinolin-2-yl)pentanamide **(24)**
   ¹H NMR (400 MHz, MeOD) δ 7.50 - 7.57 (m, 2H), 7.37 - 7.48 (m, 6H), 7.25 - 7.37 (m, 6H), 7.17 - 7.24 (m, 4H), 4.26 - 4.50 (m, 2H), 4.34 (dd, *J =* 5.00, 7.80 Hz, 1H), 3.25 - 3.75 (m, 2H), 3.37 (t, *J* = 7.28 Hz, 2H), 3.13 - 3.26 (m, 4H), 2.77 (t, *J* = 7.30 Hz, 2H), 1.47 - 1.76 (m, 4H). LRMS (ESI) (C₃₅H₃₇N₃O₂) [M + H]⁺ 532.2.
● (2R)-N-(2-phenylethyl)-2-[(2-phenylphenyl)formamido]-5-(1,2,3,4-tetrahydroisoquinolin-2-yl)pentanamide **(25)**
   LRMS (ESI) (C₃₅H₃₇N₃O₂) [M + H]⁺ 532.3.
● (2R)-N-(2-phenylethyl)-2-[(2-phenylphenyl)formamido]-5-(piperidin-1-yl)pentanamide **(27)**
   LRMS (ESI) (C₃₁H₃₇N₃O₂) [M + H]⁺ 484.1.
● (2S)-2-[(2-phenylphenyl)formamido]-N-(3-phenylpropyl)-5-(piperidin-1-yl)pentanamide **(28)**
   LRMS (ESI) (C₃₂H₃₉N₃O₂) [M + H]⁺ 498.2.
● (2S)-2-{[2-(4-methoxyphenyl)phenyl]formamido}-N-(3-phenylpropyl)-5-(piperidin-1-yl)pentanamide **(29)**
   LRMS (ESI) (C₃₃H₄₁N₃O₃) [M + H]⁺ 520.2.
● (2S)-5-(azepan-1-yl)-2-{[2-(4-methoxyphenyl)phenyl]formamido}-N-(3-phenylpropyl)pentanamide **(30)**
   LRMS (ESI) (C₃₄H₄₃N₃O₃) [M + H]⁺ 542.2.
● (2S)-N-(2-phenylethyl)-2-[(2-phenylphenyl)formamido]-5-(4-phenylpiperidin-1-yl)pentanamide **(31)**
   ¹H NMR (400 MHz, MeOD) δ 7.49 - 7.59 (m, 2H), 7.15 - 7.49 (m, 17H), 4.28 - 4.39 (m, 1H), 3.50 - 3.59 (m, 2H), 3.38 (t, *J =* 7.28 Hz, 2H), 2.96 - 3.12 (m, 4H), 2.83 - 2.95 (m, 1H), 2.78 (t, *J=* 7.40 Hz, 2H), 1.93 - 2.13 (m, 4H), 1.46 - 1.73 (m, 4H).
● (2S)-5-(4-benzylpiperidin-1-yl)-N-(2-phenylethyl)-2-[(2-phenylphenyl)formamido]pentanamide **(32)**
   ¹H NMR (400 MHz, MeOD) δ 7.48 - 7.58 (m, 2H), 7.33 - 7.48 (m, 7H), 7.25 - 7.33 (m, 4H), 7.16 - 7.25 (m, 6H), 4.26 - 4.33 (m, 1H), 3.39 (m, 2H), 3.37 (t, *J* = 7.28 Hz, 2H), 2.90 - 3.03 (m, 2H), 2.78 - 2.87 (m, 2H), 2.72 - 2.79 (t, *J* = 7.30 Hz, 2H), 2.62 (d, *J* = 6.53 Hz, 2H), 1.79 - 1.96 (m, 3H), 1.57 - 1.70 (m, 1H), 1.41 - 1.57 (m, 5H). LRMS (ESI) (C₃₈H₄₃N₃O₂) [M + H]⁺ 574.3.
● (2S)-N-(2-phenylethyl)-2-[(2-phenylphenyl)formamido]-5-[4-(piperidin-1-yl)piperidin-1-yl]pentanamide **(33)**
   ¹H NMR (400 MHz, MeOD) δ 7.50 - 7.58 (m, 2H), 7.34 - 7.49 (m, 7H), 7.25 - 7.32 (m, 2H), 7.17 - 7.25 (m, 3H), 4.28 - 4.36 (m, 1H), 3.48 - 3.69 (m, 5H), 3.37 (t, *J =* 7.28 Hz, 2H), 2.97 - 3.15 (m, 6H), 2.77 (t, *J =* 7.30 Hz, 2H), 2.33 - 2.47 (m, 2H), 2.12 - 2.28 (m, 2H), 1.75 - 2.07 (m, 5H), 1.45 - 1.72 (m, 5H). LRMS (ESI) (C₃₆H₄₆N₄O₂) [M + H]⁺ 567.3.
● (2S)-5-(4-methylpiperazin-1-yl)-N-(2-phenylethyl)-2-[(2-phenylphenyl)formamido]pentanamide **(34)**
   ¹H NMR (400 MHz, MeOD) δ 7.47 - 7.58 (m, 2H), 7.32 - 7.47 (m, 7H), 7.25 - 7.31 (m, 2H), 7.15 - 7.25 (m, 3H), 4.23 (dd, *J=* 5.77, 8.03 Hz, 1H), 3.33 - 3.46 (m, 2H), 2.76 (t, *J* = 7.30 Hz, 2H), 2.44 - 2.72 (m, 8H), 2.39 (s, 3H), 2.28 (t, *J=* 7.15 Hz, 2H), 1.39 - 1.58 (m, 2H), 1.17 - 1.37 (m, 2H). LRMS (ESI) (C₃₁H₃₈N₄O₂) [M + H]⁺ 499.3.
● (2S)-5-(4-acetylpiperazin-1-yl)-N-(2-phenylethyl)-2-[(2-phenylphenyl)formamido]pentanamide **(35)**
   ¹H NMR (400 MHz, MeOD) δ 7.47 - 7.58 (m, 2H), 7.31 - 7.47 (m, 7H), 7.24 - 7.31 (m, 2H), 7.15 - 7.24 (m, 3H), 4.24 (dd, *J* = 5.52, 8.28 Hz, 1H), 3.46 - 3.63 (m, 4H), 3.32 - 3.46 (m, 2H), 2.76 (t, *J* = 7.50 Hz, 2H), 2.42 - 2.50 (m, 2H), 2.39 (t, *J* = 5.14 Hz, 2H), 2.31 (t, J = 7.28 Hz, 2H), 2.09 (s, 3H), 1.41 - 1.61 (m, 2H), 1.20 - 1.37 (m, 2H). LRMS (ESI) (C₃₂H₃₈N₄O₃) [M + H]⁺ 527.2.
● (2S)-5-(morpholin-4-yl)-N-(2-phenylethyl)-2-[(2-phenylphenyl)formamido]pentanamide (36)
   LRMS (ESI) (C₃₀H₃₅N₃O₃) [M + H]⁺ 486.2.
● (2S)-5-[4-(2-oxo-2,3-dihydro-1H-indol-1-yl)piperidin-1-yl]-N-(2-phenylethyl)-2-[(2-phenylphenyl)formamido]pentanamide (37)
   LRMS (ESI) (C₃₉H₄₂N₄O₃) [M + H]⁺ 615.2.
● (2S)-5-[(1-benzylpiperidin-4-yl)(methyl)amino]-N-(2-phenylethyl)-2-[(2-phenylphenyl)formamido]pentanamide (38)
   LRMS (ESI) (C₃₉H₄₆N₄O₂) [M + H]⁺ 603.3.
● (2S)-5-(4-benzoylpiperazin-1-yl)-N-(2-phenylethyl)-2-[(2-phenylphenyl)formamido]pentanamide (39)
   ¹H NMR (400 MHz, MeOD) δ 7.30 - 7.55 (m, 14H), 7.23 - 7.30 (m, 2H), 7.15 - 7.23 (m, 3H), 4.26 (dd, J = 5.52, 8.28 Hz, 1H), 3.66 - 3.86 (m, 2H), 3.33 - 3.52 (m, 4H), 2.76 (t, J = 7.50 Hz, 2H), 2.40 - 2.63 (m, 4H), 2.36 (t, J = 6.65 Hz, 2H), 1.42 - 1.62 (m, 2H), 1.20 - 1.40 (m, 2H). LRMS (ESI) (C₃₇H₄₀N₄O₃) [M + H]⁺ 589.2.
● (2S)-5-(4-benzylpiperidin-1-yl)-N-(2-phenylethyl)-2-(phenylformamido)pentanamide (40)
   LRMS (ESI) (C₃₂H₃₉N₃O₂) [M + H]⁺ 498.2.
● (2S)-5-(4-benzylpiperidin-1-yl)-2-[(2-chlorophenyl)formamido]-N-(2-phenylethyl)pentanamide (**41**)
   LRMS (ESI) (C₃₂H₃₈ClN₃O₂) [M + H]⁺ 532.2.
● (2S)-5-(4-benzylpiperidin-1-yl)-N-(2-phenylethyl)-2-(quinolin-2-ylformamido)pentanamide (**42**)
   LRMS (ESI) (C₃₅H₄₀N₄O₂) [M + H]⁺ 549.2.
● (2S)-5-(4-benzylpiperidin-1-yl)-2-[(3-chlorophenyl)formamido]-N-(2-phenylethyl)pentanamide (**43**)
   LRMS (ESI) (C₃₂H₃₈ClN₃O₂) [M + H]⁺ 532.2.
● (2S)-2-(adamantan-1-ylformamido)-5-(4-benzylpiperidin-1-yl)-N-(2-phenylethyl)pentanamide (**44**)
   LRMS (ESI) (C₃₆H₄₉N₃O₂) [M + H]⁺ 556.4.
● (2S)-5-(4-benzylpiperidin-1-yl)-2-(3,3-diphenylprop-2-enamido)-N-(2-phenylethyl)pentanamide (**45**)
   LRMS (ESI) (C₄₀H₄₅N₃O₂) [M + H]⁺ 600.4.
● (2S)-5-(4-benzylpiperidin-1-yl)-N-(2-phenylethyl)-2-[4-(phenylformamido)butanamido]pentanamide (**46**)
   LRMS (ESI) (C₃₆H₄₆N₄O₃) [M + H]⁺ 583

Compounds **48-70** can be prepared by analogous variations of these synthetic procedures.

### Example 3 : Synthesis of compound 47

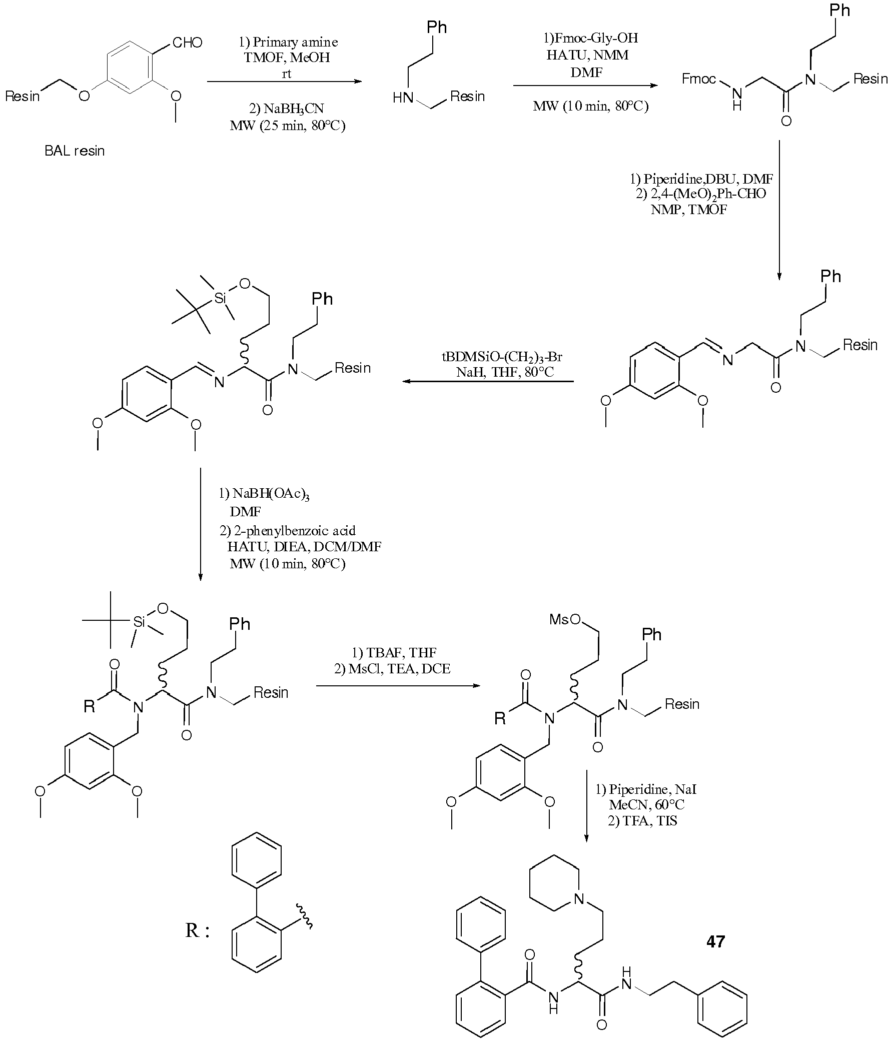

To a suspension of BAL resin (127 mg, 0.94 mmol/g) in a mixture TMOF/Methanol (2 mL:0.8 mL) was added phenethylamine (10 eq., 151 µL), and the reaction mixture was stirred at room temperature for 90 minutes. Then, sodium cyanotrihydroborate (10eq., 76 mg) was added and the reaction mixture was irradiated by microwave at 80°C for 25 minutes and the mixture was stirred overnight at room temperature. After filtration and washes (2 x 1 min MeOH, 2 x 1 min DCM, 2 x 1 min DMF), a mixture of Fmoc-Gly-OH (5 eq., 179 mg), HATU (4.8 eq., 220 mg) and DIEA (10 eq., 199 µL) in DMF (2.5 mL) was added, and the mixture was irradiated by microwave at 80°C for 10 minutes. After filtration, this operation was repeated once. After filtration and washes (2 x 1 min DMF , 2 x 1 min DCM, 2 x 1 min MeOH, 2 x 1 min DCM, 2 x 1 min DMF), resin was treated twice with a solution of DBU/piperidine (2%/2% v/v in DMF (2.5 mL)), for 1 and 20 minutes respectively. After filtration and washes (2 x 1 min DMF , 2 x 1 min DCM, 2 x 1 min MeOH, 2 x 1 min DCM, 2 x 1 min DMF), Fmoc titration by HPLC led to estimate the resin loading about 0.48 mmol/g. Then, resin was treated with a solution of 2,4-dimethoxybenzaldehyde (20 eq., 202 mg) in N-methylpyrrolidone/TMOF [4:1] (2.5 mL) and stirred overnight at room temperature. Resin was filtrated and washed (2 x 1 min DMF, 2 x 1 min DCM, 2 x 1 min Et20). To a suspension of resin in dry THF (2 mL) were added (3-Bromopropoxy)tertButyldimethylsilane (10 eq., 141 µL), sodium hydride (60% in mineral oil, 10 eq., 23 mg), and the reaction mixture was stirred at 80°C for 6 hours. Resin was then filtrated and washed (2 x 1 min MeOH, 2 x 1 min DCM, 2 x 1 min DMF). To a suspension of resin in DMF was added sodium triacetoxyhydroborate (10 eq., 129 mg), and the reaction mixture was stirred overnight at room temperature. After filtration and washes (2 x 1 min DMF/MeOH [1:1] , 2 x 1 min DCM, 2 x 1 min MeOH, 2 x 1 min DCM), resin was treated with 2-phenylbenzoic acid (5 eq., 60 mg) in presence of HATU (4.8 eq., 220 mg), DIEA (10 eq., 101 µL) in DCM/DMF (2 mL / 0.5 mL), and irradiated by microwave at 80°C for 25 minutes. After filtration, this operation was repeated once. After filtration and washes (2 x 1 min DCM, 2 x 1 min MeOH, 2 x 1 min DCM), resin was treated with a solution of TBAF (1N in THF (1.5 mL) and stirred overnight at room temperature. After filtration and washes (3 x 1 min DCM, 3 x 1 min DCE), resin was then treated with a solution of mesyl chloride (10 eq., 47 µL) and TEA (20 eq., 170 µL) in DCE for 7 hours at room temperature. After filtration and washes (2 x 1 min DCE , 2 x 1 min DCM, 2 x 1 min MeOH, 2 x 1 min DCM, 2 x 1 min Et2O), resin was treated with piperidine (20 eq., 120 µL) in MeCN in the presence of catalytic amount of sodium iodine (1 mg) and stirred overnight at 60°C. Cleavage of the resin was performed in a mixture TFA/TIS (97:3, 1.5 mL) at room temperature for 45 minutes. After filtration and evaporation, the resulting slurry was purified by reverse phase LPLC (15% to 100% MeOH in water with 0.05% TFA, 45 min, 30 mL/min, column : Simply connect C18 from AIT (40-60 µm, 50g)) and purified fractions were lyophilized to give expected racemic N-(2-phenylethyl)-2-(phenylformamido)-5-(piperidin-1-yl)pentanamide (47) as trifluoroacetate salt (8 mg, 20% yield).

¹H NMR (400 MHz, MeOD) δ 7.85 - 7.88 (m, 2H), 7.53 - 7.60 (m, 1H), 7.44 - 7.52 (m, 2H), 7.13 - 7.25 (m, 5H), 4.52 - 4.58 (m, 1H), 3.42 - 3.52 (m, 4H), 3.06 - 3.15 (m, 2H), 2.06 - 2.95 (m, 4H), 1.70 - 1.98 (m, 8H), 1.45 - 1.53 (m, 1H), 1.28 - 1.35 (m, 1H). HRMS (ESI) (C₂₅H₃₄N₃O₂) [M + H]⁺ 408.2659.

### Example 4 : Pharmacological data

### Materials and methods

### Cloning of hNPFF2 and hNPFF1-

The cDNA encoding the full-length human NPFFR1 and human NPFFR2 were subcloned into the pcDNA3 expression vector (Invitrogen, Cergy Pontoise, France) using standard molecular biology methods. A sequence encoding a signal sequence and a Flag epitope was fused to the 5' coding sequence of both hNPFFR1 and hNPFFR2 cDNA according to (Guan et al., 1992) resulting in a SF-hNPFFR1 and SF-hNPFFR2 constructs. These contructs were stably transfected into CHO cells.

### Cell membrane preparations and receptor binding assays-

Membranes from CHO cells stably expressing SF-hNPFFR1 or SF-hNPFFR2 were prepared as described (Becker et al., 1999). SF-hNPFFR1 or SF-hNPFFR2 membranes (10µg) were incubated for 30 min at 25 °C in a final volume of 0.5 ml containing 50 mM HEPES pH 7.4, 1 mM CaCl₂, 1 mM MgCl₂, 0.1% bovine serum albumin, 10 nM and 3 nM [³H]-FFRFamide (CEA, Paris; specific activity, 13,6 Ci/mmol) for SF-hNPFFR1 and SF-hNPFFR1, respectively and the ligands to be tested. Non-specific binding was determined in presence of 1 µM neuropeptide FF for SF-hNPFFR2 and 1 µM neuropeptide VF for SF -hNPFFR1. Typical total and nonspecific binding were 1200 and 200 dpm, respectively for SF-hNPFFR1 and SF-hNPFFR2. Incubation mixtures were rapidly filtered and washed with 50 mM HEPES pH 7.4, 1 mM CaCl₂, 1 mM MgCl₂, on 96 wells GF/B unifilter (Perkin Elmer). Unifilter plates where then dryied for 1hr at 65°C. Bound radioactivity was determined by scintillation counting with 30 µl of scintillation cocktail (O-scint, Perkin Elmer) per well on a Topcount scintillation apparatus (Perkin Elmer).

### Cellular assays-

### [³⁵S]GTPγS binding measurement

For [³⁵S]GTPγS binding experiments, SF-hNPFFR1 or SF-hNPFFR2 membrane proteins (5 µg) were incubated for 45 min at 30 °C in a final volume of 0.5 ml containing 20 mM HEPES pH 7.4, 100 mM NaCl, 3 mM MgCl₂, 10 µM GDP, 10 µg/ml Saponin, 0.1 nM [³⁵S]GTPγS and ligands. Non-specific binding was determined in the presence of 10 µM GTPγS. Incubation mixtures were rapidly filtered and washed with 10 mM HEPES pH 7.4, 0,5 M NaCl, 1,5 mM MgCl₂, on 96 wells GF/B unifilter (Perkin Elmer). Unifilter plates where then dryied for 1hr at 65°C. Bound radioactivity was determined by scintillation counting with 30 µl of scintillation cocktail (O-scint, Perkin Elmer) per well on a Topcount scintillation apparatus (Perkin Elmer).

### cAMP measurements

Functional coupling of SF-hNPFFR1 and SF-hNPFFR2 to adenylyl cyclase was assessed by measuring the inhibitory effects of agonist/antagonist and related compounds on forskolin-stimulated cAMP accumulation in CHO cells stably expressing both receptors as previously described (Gicquiaux et al., 2001) with minor modifications. Briefly, 80% confluent CHO cells expressing SF-hNPFFR1 or SF-hNPFFR2 were harvested and seeded in 96-well plates at an initial density of 10,000 cells per well. After 1-2 days of culture (90% confluency), cells were washed with 0.2 ml of PBS (in mM: 137 NaCl, 2.7 KCl, 0.9 CaCl₂, 0.5 MgCl₂, 6.5 Na₂HPO₄, 1.5 KH₂PO₄, pH 7.2), and then incubated at 37°C for 10 min with 0.2 ml assay buffer (in mM: 150 NaCl, 5 KCl, 2.5 CaCl₂, 1.2 KH₂PO₄, 1.2 MgSO₄, 25 NaHCO₃, 10 HEPES, 10 mg/ml BSA, pH 7.4). After removal of buffer, 0.06 ml of fresh buffer containing 1 mM 3-isobutyl-1-methyl-xanthine (IBMX) was added for 10 min and cells were exposed to 10 µM forskolin without and with compounds (10⁻¹⁰-10⁻⁵ M) for 10 min at 37 °C in a final volume of 0.1 ml. The reaction was stopped by addition of 0.04 ml volume ice-cold 0.5 M HCl and cells were frozen (-80°C 1 hour) disrupted by sonication and suspensions were centrifuged at 2,000 × *g* for 15 min. The resulting supernatants were stored at -20°C until determination of cAMP by radioimmunoassay as described (Cailla et al., 1973). For antagonist evaluation, dose response curves of agonists (NPVF and NPFF for hNPFFR1 and hNPFFR2, respectively) were performed in the presence of fixed concentrations of antagonist. Shild analysis was used to calculate the pA₂ of the antagonists (i.e., the -log of the value required to double the agonist's EC50).

### In vivo assays

### Fentanyl-induced hyperalgesia in rats

On Day 0 (D0), fentanyl (80 µg/kg) injections were performed every 15 min for 1 h, resulting in a total dose of 320 µg/kg and nociceptive threshold was measured by the paw pressure test as described in Laboureyras et al., 2009. Compound 26 was dissolved in saline and administered, either by subcutaneous injection (Fig 1) or by oral route (Fig 2) 30 min. before fentanyl. Doses of compound 26 used were : 0.05, 0.1 and 0.5 mg/kg or 0.3, 1 and 3 mg/kg for subcutaneous and oral administrations (respectively). Calculation corresponding to area under curve (AUC) of hyperalgesia index (HI) and statistical analysis were performed as described in Laboureyras et al., 2009.

### Fentanyl-induced hyperalgesia followed by carrageenan injection in rats

On Day 0 (D0), fentanyl (4 X 100 µg) was administered as described above. Nociceptive threshold was measured (Laboureyras et al., 2009) during twelve days on both left (inflamed) and right (non-inflamed) hind paw. Carrageenan (0.2 ml of a 1% carrageenan solution in saline) was injected on day thirteen (D13) into the plantar aspect of the left hind paw as described in Laboureyras et al., 2009, and nociceptive threshold was measured on both left and right hind paw during eight days after the injection. Compound 26 (0.5 mg/kg, s.c.) was administered 30 min before fentanyl 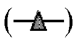 . Calculation of hyperalgesia index (HI) and statistical analysis were performed as described in Laboureyras et al., 2009.

### In vitro Results

Compound 26 displayed a good affinity for NPFFR1 and very low affinity for NPFFR2 (table 1). When tested alone (up to 50 µM) compound 26 did not stimulate the binding of [³⁵S]GTPgS in membrane from cells expressing hNPFFR1 and did not decrease the forskolin-stimulated cAMP accumulation in cells stably expressing this receptor. Its capability to antagonize the activity of NPVF at hNFFR1 was further evaluated in cAMP assay. pA₂ value of compound 26 for hNPFFR1 together with pA₂ values for the reference compound RF9 for hNPFFR1 and hNPFFR2 are reported in table 2. These data demonstrate that in vitro compound 26 displays antagonist activity at hNPFFR1.

**Table 1: Binding assay with both receptors NPFFR1 & 2^{a}**

| Cpd | hNPFF1 | | | hNPFF2 | | |
|---|---|---|---|---|---|---|
| | % inh. 5 µM^{b} | % inh. 0.5 µM^{b} | Ki NPFF1 (nM)^{b} | % inh. 5 µM^{b} | % inh. 0.5 µM^{b} | Ki NPFF2 (nM)^{b} |
| **1** | | | 240 ± 38 | | | 2300 ± 66 |
| **2** | | | 480 ± 33 | | | 910 ± 94 |
| **3** | 61 ± 1 | 29 ± 2 | nd | 24 ± 6 | 15 ± 15 | nd |
| **4** | 23 ± 0 | 14 ± 1 | nd | 7 ± 15 | 2 ± 6 | nd |
| **5** | | | 56 ± 11 | | | 1300 ±590 |
| **6** | | | 210 ± 39 | | | 720 ±59 |
| **7** | | | 22 ± 4 | | | 605 ±180 |
| **8** | | | 23 ± 4 | | | 103 ±44 |
| **9** | 60 ± 3 | 21 ± 4 | nd | 45 ± 0 | 9 ± 1 | nd |
| **10** | | | 30 ± 2 | 40 ± 2 | 11 ± 20 | nd |
| **11** | 37 ± 1 | 49 ± 37 | nd | 26 ± 8 | 8 ± 3 | nd |
| **12** | 74 ± 2 | 32 ± 1 | nd | 64 ± 3 | 25 ± 5 | nd |
| **13** | | | 59 ± 14 | 67 ± 4 | 23 ± 17 | nd |
| **14** | | | 45 ± 4 | 54 ± 3 | 16 ± 2 | nd |
| **15** | | | 680 ± 160 | | | 1100 ±510 |
| **16** | 50 ± 10 | 9 ± 4 | nd | 47 ± 1 | 6 ± 13 | nd |
| **17** | 65 ± 6 | 17 ± 5 | nd | 48 ± 3 | 18 ± 2 | nd |
| **18** | 45 ± 2 | 8 ± 3 | nd | 40 ± 3 | 8 ± 5 | nd |
| **19** | 60 ± 2 | 16 ± 5 | nd | 39 ± 7 | 0 ± 1 | nd |
| **20** | 51 ± 2 | 14 ± 7 | nd | 39 ± 1 | 11 ± 3 | nd |
| **21** | 58 ± 14 | 12 ± 10 | nd | 47 ± 0 | 0 ± 2 | nd |
| **22** | 50 ± 2 | 7 ± 6 | nd | 39 ± 2 | 6 ± 0 | nd |
| **23** | | | 103 ± 15 | 35 ± 8 | 22 ± 3 | nd |
| **24** | | | 500 ± 10 | | | 320 ± 10 |
| **25** | 51 ± 14 | 14 ± 1 | nd | 16 ± 5 | 0 ± 8 | nd |
| **26** | | | 65 ± 1 | | | > 13 µM |
| **27** | | | 179 ± 64 | 13 ± 8 | 0 ± 3 | nd |
| **28** | 41 ± 2 | 16 ± 25 | | 17 ± 0 | 0 ± 2 | nd |
| **29** | | | 170 ± 70 | 23 ± 5 | 0 ± 27 | nd |
| **30** | | | 64 ± 32 | | | 76 ± 18 |
| **31** | | | 485 ± 35 | 45 ± 7 | 0 ± 7 | nd |
| **32** | | | 400 ± 71 | | | 34 ± 12 |
| **33** | | | 71 ± 11 | 55 ± 15 | 13 ± 11 | nd |
| **34** | | | 232 ± 17 | 12 ± 5 | 0 ± 21 | nd |
| **35** | | | 319 ± 13 | 0 ± 11 | 0 ± 3 | nd |
| **36** | 25 ± 10 | 10 ± 3 | nd | 25 ± 2 | 2 ± 0 | nd |
| **37** | 42 ± 3 | 21 ± 6 | nd | 45 ± 0 | 0 ± 4 | nd |
| **38** | | | 85 ± 20 | | | 40 ±23 |
| **39** | | | 187 ± 9 | 17 ± 0 | 0 ± 7 | nd |
| **40** | 32 ± 1 | 19 ± 3 | nd | 19 ± 1 | 5 ± 3 | nd |
| **41** | 54 ± 3 | 0 ± 3 | nd | 41 ± 3 | 0 ± 3 | nd |
| **42** | 72 ± 11 | 11 ± 15 | nd | 47 ± 0 | 4 ± 10 | nd |
| **43** | | | 441 ± 80 | | | 147 ± 2 |
| **44** | | | 603 ±139 | | | 188 ± 6 |
| **45** | 12 ± 2 | 10 ± 18 | nd | 25 ± 19 | 23 ± 25 | nd |
| **46** | 59^{d} ± 1 | 35^{e} ± 1 | nd | 39^{d} ± 13 | 4^{e} ± 17 | nd |

| | | | | | | |
|---|---|---|---|---|---|---|
| ^{a}[³H]-FFRF-NH₂ was used as radioligand for both receptors. ^{b} Mean value of at least two experiments ^{c}nd : not determined ^{d}% Inhibition at 7 µM^{e} % Inhibition at 0.7 µM. | | | | | | |

**Table 2: . pA₂ value of Compound 26 and reference compound for NPFF receptors**

| Compounds | pA₂ value for hNPFFR1 | pA₂ for hNPFFR2 |
|---|---|---|
| RF9 | 6.47 | 6,65 |
| Compound 26 | 6.01 | -* |

| | | |
|---|---|---|
| * the pA₂ value of compound 26 for hNPFF2 was not evaluated due to the very low affinity of this compound for this receptor (see table 1). S.E.M. were below 15%. | | |

### In vivo Results

### Preventive effect of compound 26 on fentanyl-induced hyperalgesia in rats

As previously described in Laboureyras et al., 2009, fentanyl administration induced analgesia that was followed by hyperalgesia for several days (Fig. 1 and 2). When administered 30 min before fentanyl, compound 26 administered either subcutaneously (Fig. 1) or orally (Fig. 2) dose-dependently prevented the development of fentanyl-induced hyperalgesia. Hyperalgesia was completely abolished when compound 26 was co-administered with fentanyl at 0.5 mg/kg subcutaneously and 3 mg/kg orally (P < 0.001, Newman-Keuls test). These results clearly show that pharmacological blockade of NPFF1 receptor with compound 26 efficiently prevents long lasting secondary hyperalgesia induced by fentanyl.

### Compound 26 prevents the increase in inflammatory hyperalgesia induced by prior fentanyl administration.

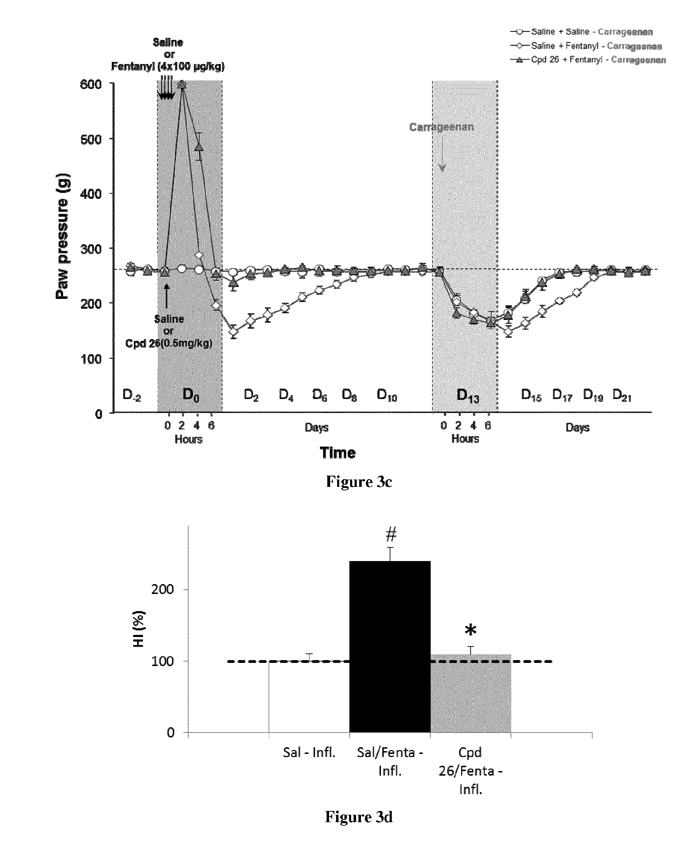

As described above, administration of fentanyl in rats induced analgesia and secondary hyperalgesia on the following days that was prevented by co-administration of compound 26 with fentanyl (Fig. 1, Fig. 2, Fig. 3). In the same animals, when an inflammation was induced thirteen days after fentanyl administration by injection of carrageenan in the left hind paw, the development of a hyperalgesia was greatly enhanced compared to animals that were treated with saline at Do as indicated by the statistically significant increase of hyperalgesia index from 100% (saline - carrageenan animals) to 200% (fentanyl - carrageenan animals; Fig. 3b, d). This effect was observed when nociceptive threshold was measured on both hind paws (Fig. 3 ; P < 0.001, Newman-Keuls test). Finally, the increase in inflammatory hyperalgesia induced by prior fentanyl injection was completely prevented by co-administration of compound 26 with fentanyl (P < 0.001 Newman-Keuls test). These results indicate that fentanyl induced long-term pain hypersensitivity that led to exaggerated hyperalgesia induced by subsequent inflammation and that this effect was completely blocked by pharmacological blockade of NPFF1 receptor with compound 26.

### References

- Becker, J. A., Wallace, A., Garzon, A., Ingallinella, P., Bianchi, E., Cortese, R., Simonin, F., Kieffer, B. L. & Pessi, A. (1999) J. Biol. Chem. 274, 27513-27522.
- Cailla H.L., Racine-Weisbuch M.S., Delaage M.A. (1973). Adenosine 3',5' cyclic monophosphate assay at 10-15 mole level. Anal Biochem. 56, 394-407.
- Gicquiaux, H., Lecat, S., Gaire, M., Dieterlen, A., Mely, Y., Takeda, K., Bucher, B., and Galzi, J.-L. (2002) Rapid Internalization and Recycling of the Human Neuropeptide Y Y1 Receptor. J. Biol. Chem. 277, 6645-6655.
- Guan, X. M., Kobilka, T. S. & Kobilka, B. K. (1992) J Biol Chem 267, 21995-21998.
- Laboureyras E., Chateauraynaud J., Richebé P., Simonnet G. (2009). Long-term pain vulnerability after surgery in rats: prevention by nefopam, an analgesic with antihyperalgesic properties. Anesth Analg. 109, 623-631

## Claims

1. A compound that has the following general formula (I): 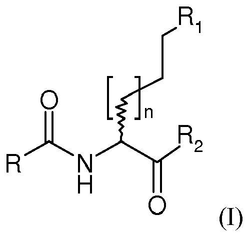 wherein:
- n is 1 or 2;
- R is a (C₁-C₄)alkyl, (C₂-C₄)alkenyl, (C₆-C₁₄)aryl, heteroaryl, aralkyl, heteroaralkyl or (C₃-C₁₀)cycloalkyl group, wherein said group is optionally substituted by at least one substituent selected from a halogen atom, a (C₁-C₄)alkyl, (C₁-C₄)alkoxy, thioalkyl, aryl, aralkyl or heteroaryl group, wherein said substituent group is optionally substituted by at least one group selected from: -COOH, halogen, (C₁-C₄)alkyl, (C₁-C₄)alkoxy, perhalogeno(C₁-C₄)alkyl, hydroxyl, NO₂ and NH₂;
- R₁ represents an amino group selected from:
(i) 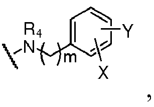 wherein m is 1, 2 or 3, R₄ is a hydrogen atom or a (C₁-C₄)alkyl group, X and Y independently represent a hydrogen atom, a halogen atom, a hydroxyl, a (C₁-C₄)alkoxy, a perhalogeno(C₁-C₄)alkyl group, an acetyl group (-COCH₃), or an amino group that is mono- or bi-substituted by an alkyl or heterocycle group, or X and Y may form together an -O-(CH₂)ₚ-O- group, wherein p is 1, 2 or 3;
(ii) 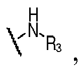 wherein R₃ is a hydrogen atom, a (C₁-C₄)alkyl, aralkyl or (C₅-C₈)cycloalkyl group;
(iii) 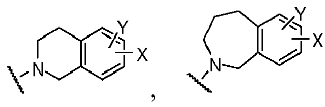 or 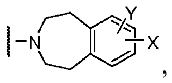 wherein X and Y independently represent a hydrogen atom, a halogen atom, a hydroxyl, a (C₁-C₄)alkyl, a (C₁-C₄)alkoxy, a perhalogeno(C₁-C₄)alkyl group, an acetyl group (-COCH₃), or an amino group that is mono- or bi-substituted by an alkyl or heterocycle group, or X and Y may form together an -O-(CH₂)ₚ-O- group, wherein p is 1, 2 or 3;
(iv) 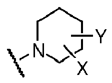 or 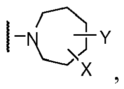 wherein X represents a hydrogen atom; a halogen atom; a hydroxyl group; an acetyl group (-COCH₃); an aryl; aryloxy; aralkyl; N-heterocycle; aryl-NH-; aralkyl-NH-; aroyl-NH; -NHCOCH₃ ;-NHCHO group; a 2-oxoindoline group; a -CO-R₅ group, wherein R₅ is an amino group that is mono- or bi-substituted by an alkyl or heterocycle group; a (C₁-C₄)alkyl group; a perhalogeno(C₁-C₄)alkyl group; a (C₁-C₄)alkoxy group; -NR₆COR₇, wherein R₆ is a hydrogen atom or a (C₁-C₄)alkyl group, and R₇ is a hydrogen atom, a (C₁-C₄)alkyl, aryl, or aralkyl group; a -ZR₈R₉ group, wherein Z is N or CH, R₈ is an aryl group and R₉ is a hydrogen atom, an aryl or (C₁-C₄)alkyl group; and Y is a hydrogen atom or a hydroxyl, (C₁-C₄)alkoxy,-NHCHO or NHCOCH₃ group;
(v) 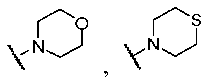 or 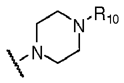 wherein R₁₀ is a hydrogen atom, a (C₁-C₄)alkyl group or a -CO-R₁₁ group wherein R₁₁ is a hydrogen atom or a (C₁-C₄)alkyl, aryl or heteroaryl group;
(vi) 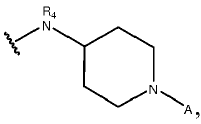 wherein R₄ is as defined above and A is a hydrogen atom or an alkyl or aralkyl group;
(vii) 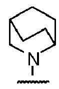 or 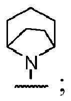 and
(viii) 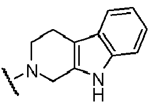 or 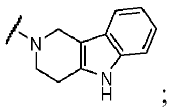
- R₂ represents an amino group selected from:
(i) 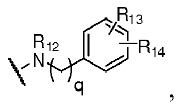 wherein q is 1, 2 or 3, R₁₂ is a hydrogen atom or a (C₁-C₄)alkyl group, R₁₃ is a hydrogen atom, a halogen atom, a (C₁-C₄)alkyl, a perhalogeno(C₁-C₄)alkyl, a (C₁-C₄)alkoxy group, an hydroxyl group, an acetyl group (-COCH₃), or an amino group mono- or bi-substituted by an alkyl or heterocycle group, R₁₄ is a hydrogen atom, a halogen atom, a (C₁-C₄)alkyl, a perhalogeno(C₁-C₄)alkyl, a (C₁-C₄)alkoxy group or an amino group that is mono- or bi-substituted by an alkyl or heterocycle group, and R₁₃ and R₁₄ may form together an -O-(CH₂) ᵣ-O- group, wherein r is 1, 2 or 3;
(ii) 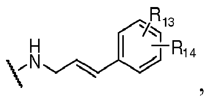 wherein R₁₃ and R₁₄ are as defined above;
(iii) 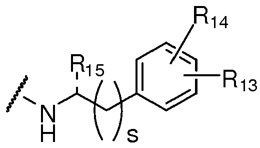 or 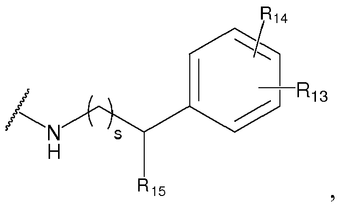 wherein R₁₃ and R₁₄ are as defined above, R₁₅ is a hydrogen atom or a (C₁-C₄)alkyl group, and s is 0 or 1;
(iv) 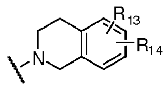 or 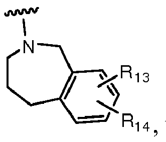 wherein R₁₃ and R₁₄ are as defined above;
(v) 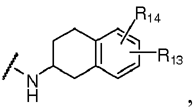 wherein R₁₃ and R₁₄ are as defined above; and
(vi) 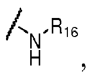 wherein R₁₆ is a hydrogen atom or a (C₁-C₄)alkyl group.
(vii) 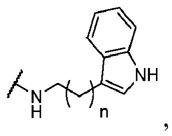 wherein n is 0 or 1.

2. The compound according to claim 1, wherein R represents a group selected from:
(i) 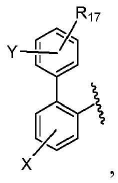 wherein R₁₇ is a hydrogen atom, a halogen atom or a (C₁-C₄)alkyl, perhalogeno(C₁-C₄)alkyl, (C₁-C₄)thioalkyl or (C₁-C₄)alkoxy group; X and Y independently represent a hydrogen atom, a halogen atom, a hydroxyl, (C₁-C₄)alkoxy, perhalogeno(C₁-C₄)alkyl group or an amino group that is mono- or bi-substituted by an alkyl or heterocycle group, or X and Y may form together an -O-(CH₂)ₚ-O- group, wherein p is 1, 2 or 3;
(ii) 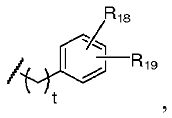 wherein t is 0, 1, 2, 3, 4, or 5 (preferably 0), R₁₈ is a hydrogen atom, a halogen atom, a hydroxyl, (C₁-C₄)alkyl, (C₁-C₄)alkoxy, (C₁-C₄)thioalkyl or perhalogeno(C₁-C₄)alkyl group, an amino group mono- or bi-substituted by an alkyl or heterocycle group, an NO₂, aryl, heteroaryl or aralkyl group wherein said aryl, heteroaryl or aralkyl group is optionally substituted by at least one group selected from: a halogen atom, a hydroxyl, (C₁-C₄)alkyl, (C₁-C₄)alkoxy, (C₁-C₄)thioalkyl, perhalogeno(C₁-C₄)alkyl or COOH group, R₁₉ is a hydrogen atom, a halogen atom, a hydroxyl, (C₁-C₄)alkyl, (C₁-C₄)alkoxy, (C₁-C₄)thioalkyl or perhalogeno(C₁-C₄)alkyl group, or an amino group mono- or bi-substituted by an alkyl or heterocycle group;
(iii) 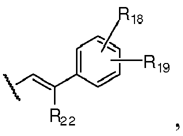 wherein R₂₂ is a hydrogen atom or a (C₁-C₄)alkyl group, and R₁₈ and R₁₉, independently, are as defined above,
(iv) 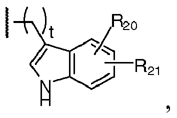 wherein t is 0, 1, 2, 3, 4, or 5, and R₂₀ is a hydrogen atom, a halogen atom, a (C₁-C₄)alkyl, perhalogeno(C₁-C₄)alkyl, (C₁-C₄)alkoxy; (C₁-C₄)thioalkyl or aryl group, or an amino group mono- or bi-substituted by an alkyl or heterocycle group, R₂₁ is a hydrogen atom, a halogen atom, a (C₁-C₄)alkyl, perhalogeno(C₁-C₄)alkyl, (C₁-C₄)alkoxy or (C₁-C₄)thioalkyl group, or an amino group mono- or bi-substituted by an alkyl or heterocycle group, wherein R₂₀ and R₂₁ together may also form an -O-(CH₂)ᵤ-O- ring attached to the phenyl group, wherein u is 1, 2 or 3;
(v) 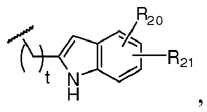 wherein t, R₂₀ and R₂₁ are as defined above;
(vi) 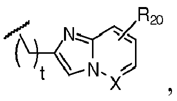 wherein t and R₂₀ are as defined above, and X is N or CH;
(vii) 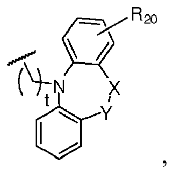 wherein t and R₂₀ are as defined above, X-Y is CH₂-CH₂, CO-CH₂, CO-N(alkyl) or CH=CH;
(viii) 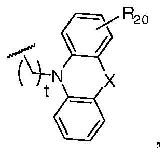 wherein t and R₂₀ are as defined above, X is S, O, NH, N(alkyl) or CH₂,
(ix) benzofuran, more specifically 1-benzofuran-2-yl, and
(x)

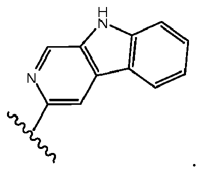

3. The compound according to claim 1 or 2, wherein R represents a group selected from:
- 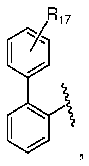 wherein R₁₇ is a hydrogen atom, a halogen atom, a (C₁-C₄)alkyl, perhalogeno(C₁-C₄)alkyl, (C₁-C₄)thioalkyl or (C₁-C₄)alkoxy group, preferably R₁₇ is a hydrogen atom or a (C₁-C₄)alkoxy group, in particular OCH₃;
- 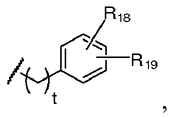 wherein t is 0, 1, 2, 3, 4, or 5 (preferably 0), R₁₈ is a hydrogen atom, a halogen atom or a hydroxyl or (C₁-C₄)alkoxy group, and R₁₉ is a hydrogen atom, a halogen atom or a hydroxyl or (C₁-C₄)alkoxy group;
- a cycloalkyl group, preferably an adamantyl ring: 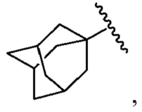 optionally substituted by -COOH, halogen, (C₁-C₄)alkyl, (C₁-C₄)alkoxy, perhalogeno(C₁-C₄)alkyl, hydroxyl, NO₂ or NH₂, in particular by at least one halogen atom or (C₁-C₄)alkyl or (C₁-C₄)alkoxy group;
- a heteroaryl group selected from a pyridine, furan, benzofuran, quinoline, indole and benzimidazole group, wherein said group is optionally substituted by at least one group selected from: -COOH, halogen, (C₁-C₄)alkyl, (C₁-C₄)alkoxy, perhalogeno(C₁-C₄)alkyl, hydroxyl, NO₂ and NH₂, in particular by at least one halogen atom or a (C₁-C₄)alkyl or (C₁-C₄)alkoxy group;
- 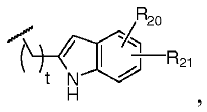 wherein t, R₂₀ and R₂₁ are as defined above, in particular t is 0, 1 or 2, and/or in particular R₂₀ and R₂₁, identical or different, are a hydrogen atom, a halogen atom or a (C₁-C₄)alkyl, perhalogeno(C₁-C₄)alkyl, or (C₁-C₄)alkoxy group;
- 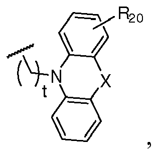 wherein t and R₂₀ are as defined above, X is S, O, NH, N(alkyl), or CH₂, in particular t is 1 or 2, and/or in particular R₂₀ is a hydrogen atom, a halogen atom or a (C₁-C₄)alkyl, perhalogeno(C₁-C₄)alkyl or (C₁-C₄)alkoxy group, and/or in particular X is S.

4. The compound according to one of claims 1-3, wherein R₁ represents an amino group selected from:
(i) 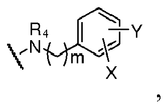 wherein m, R₄, X and Y are as defined above, in particular m is 1 or 2, R₄ is a hydrogen atom or a (C₁-C₄)alkyl group (such as methyl), X and Y independently represent a hydrogen atom, a halogen atom or a (C₁-C₄)alkoxy group (such as methoxy);
(ii) 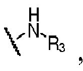 wherein R₃ is as defined above, in particular R₃ is a hydrogen atom or a (C₁-C₄)alkyl or (C₅-C₈)cycloalkyl group;
(iii) 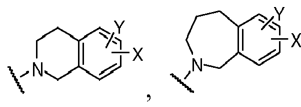 or 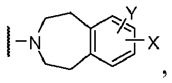 wherein X and Y are as defined above, in particular X and Y represent a hydrogen atom;
(iv) 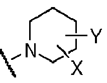 or 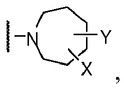 wherein X and Y are as defined above, in particular X represents a hydrogen atom, an aryl (such as phenyl), aralkyl (such as benzyl or phenethyl), N-piperidine or 2-oxoindoline group, and Y represents a hydrogen atom or a hydroxyl group;
(v) 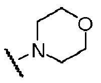 or 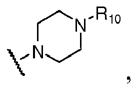 wherein R₁₀ is as defined above, in particular R₁₀ is a (C₁-C₄)alkyl group (such as methyl) or a -CO-R₁₁ group, wherein R₁₁ is a hydrogen atom or a (C₁-C₄)alkyl (such as methyl) or aryl group (such as phenyl);
(vi) 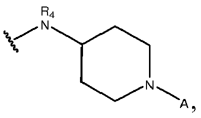 wherein R₄ and A are as defined above, in particular R₄ is a (C₁-C₄)alkyl group (such as methyl) and A is an aralkyl group (such as benzyl).

5. The compound according to one of claims 1-4, wherein wherein R₂ represents an amino group of the following formula:
- 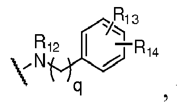 wherein q, R₁₂, R₁₃ and R₁₄ are as defined above, in particular q is 2 or 3, R₁₂ is a hydrogen atom or a (C₁-C₄)alkyl group (such as methyl), R₁₃ is a hydrogen atom, a halogen atom or a (C₁-C₄)alkoxy group (such as methoxy) and R₁₄ is a hydrogen atom.

6. The compound according to one of claims 1-5, wherein it is selected from:
1. (2S)-5-(benzylamino)-N-(2-phenylethyl)-2-[(2-phenylphenyl)formamido]pentanamide
2. (2R)-5-(benzylamino)-N-(2-phenylethyl)-2-[(2-phenylphenyl)formamido]pentanamide
3. (2S)-N-(2-phenylethyl)-5-[(2-phenylethyl)amino]-2-[(2-phenylphenyl)formamido]pentanamide
4. (2S)-5-[(2-methylpropyl)amino]-N-(2-phenylethyl)-2-[(2-phenylphenyl)formamido]pentanamide
5. (2S)-5-(benzylamino)-N-methyl-N-(2-phenylethyl)-2-[(2-phenylphenyl)formamido]pentanamide
6. (2S)-6-(benzylamino)-N-(2-phenylethyl)-2-[(2-phenylphenyl)formamido]hexanamide
7. (2R)-6-(benzylamino)-N-(2-phenylethyl)-2-[(2-phenylphenyl)formamido]hexanamide
8. (2S)-5-{[(2-methoxyphenyl)methyl]amino}-N-(2-phenylethyl)-2-[(2-phenylphenyl)formamido]pentanamide
9. (2S)-5-{[(3-methoxyphenyl)methyl]amino}-N-(2-phenylethyl)-2-[(2-phenylphenyl)formamido]pentanamide
10. (2S)-5-{[(4-methoxyphenyl)methyl]amino}-N-(2-phenylethyl)-2-[(2-phenylphenyl)formamido]pentanamide
11. (2S)-5-(cyclohexylamino)-N-(2-phenylethyl)-2-[(2-phenylphenyl)formamido]pentanamide
12. (2S)-5-{[(2-chlorophenyl)methyl]amino}-N-(2-phenylethyl)-2-[(2-phenylphenyl)formamido]pentanamide
13. (2S)-5-{[(3-chlorophenyl)methyl]amino}-N-(2-phenylethyl)-2-[(2-phenylphenyl)formamido]pentanamide
14. (2S)-5-{[(4-chlorophenyl)methyl]amino}-N-(2-phenylethyl)-2-[(2-phenylphenyl)formamido]pentanamide
15. (2S)-5-[benzyl(methyl)amino]-N-(2-phenylethyl)-2-[(2-phenylphenyl)formamido]pentanamide
16. (2S)-5-{[(2-methoxyphenyl)methyl](methyl)amino}-N-(2-phenylethyl)-2-[(2-phenylphenyl)formamido]pentanamide
17. (2S)-5-{[(3,4-dimethoxyphenyl)methyl](methyl)amino}-N-(2-phenylethyl)-2-[(2-phenylphenyl)formamido]pentanamide
18. (2S)-5-{[(3-chlorophenyl)methyl](methyl)amino}-N-(2-phenylethyl)-2-[(2-phenylphenyl)formamido]pentanamide
19. (2S)-5-{[(2-chlorophenyl)methyl](methyl)amino}-N-(2-phenylethyl)-2-[(2-phenylphenyl)formamido]pentanamide
20. (2S)-5-{[(4-chlorophenyl)methyl](methyl)amino}-N-(2-phenylethyl)-2-[(2-phenylphenyl)formamido]pentanamide
21. (2S)-5- [(4-methoxyphenyl)methyl](methyl)amino}-N-(2-phenylethyl)-2-[(2-phenylphenyl)formamido]pentanamide
22. (2S)-5-{[(3-methoxyphenyl)methyl](methyl)amino}-N-(2-phenylethyl)-2-[(2-phenylphenyl)formamido]pentanamide
23. (2S)-5-[methyl(2-phenylethyl)amino]-N-(2-phenylethyl)-2-[(2-phenylphenyl)formamido]pentanamide
24. (2S)-N-(2-phenylethyl)-2-[(2-phenylphenyl)formamido]-5-(1,2,3,4-tetrahydroisoquinolin-2-yl)pentanamide
25. (2R)-N-(2-phenylethyl)-2-[(2-phenylphenyl)formamido]-5-(1,2,3,4-tetrahydroisoquinolin-2-yl)pentanamide
26. (2S)-N-(2-phenylethyl)-2-[(2-phenylphenyl)formamido]-5-(piperidin-1-yl)pentanamide
27. (2R)-N-(2-phenylethyl)-2-[(2-phenylphenyl)formamido]-5-(piperidm-1-yl)pentanamide
28. (2S)-2-[(2-phenylphenyl)formamido]-N-(3-phenylpropyl)-5-(piperidin-1-yl)pentanamide
29. (2S)-2-{[2-(4-methoxyphenyl)phenyl]formamido}-N-(3-phenylpropyl)-5-(piperidin-1-yl)pentanamide
30. (2S)-5-(azepan-1-yl)-2-{[2-(4-methoxyphenyl)phenyl]formamido}-N-(3-phenylpropyl)pentanamide
31. (2S)-N-(2-phenylethyl)-2-[(2-phenylphenyl)formamido]-5-(4-phenylpiperidin-1-yl)pentanamide
32. (2S)-5-(4-benzylpiperidin-1-yl)-N-(2-phenylethyl)-2-[(2-phenylphenyl)formamido]pentanamide
33. (2S)-N-(2-phenylethyl)-2-[(2-phenylphenyl)formamido]-5-[4-(piperidin-1-yl)piperidin-1-yl]pentanamide
34. (2S)-5-(4-methylpiperazin-1-yl)-N-(2-phenylethyl)-2-[(2-phenylphenyl)formamido]pentanamide
35. (2S)-5-(4-acetylpiperazin-1-yl)-N-(2-phenylethyl)-2-[(2-phenylphenyl)formamido]pentanamide
36. (2S)-5-(morpholin-4-yl)-N-(2-phenylethyl)-2-[(2-phenylphenyl)formamido]pentanamide
37. (2S)-5-[4-(2-oxo-2,3-dihydro-1H-indol-1-yl)piperidin-1-yl]-N-(2-phenylethyl)-2-[(2-phenylphenyl)formamido]pentanamide
38. (2S)-5-[(1-benzylpiperidin-4-yl)(methyl)amino]-N-(2-phenylethyl)-2-[(2-phenylphenyl)formamido]pentanamide
39. (2S)-5-(4-benzoylpiperazin-1-yl)-N-(2-phenylethyl)-2-[(2-phenylphenyl)formamido]pentanamide
40. (2S)-5-(4-benzylpiperidin-1-yl)-N-(2-phenylethyl)-2-(phenylformamido)pentanamide
41. (2S)-5-(4-benzylpiperidin-1-yl)-2-[(2-chlorophenyl)formamido]-N-(2-phenylethyl)pentanamide
42. (2S)-5-(4-benzylpiperidin-1-yl)-N-(2-phenylethyl)-2-(quinolin-2-ylformamido)pentanamide
43. (2S)-5-(4-benzylpiperidin-1-yl)-2-[(3-chlorophenyl)formamido]-N-(2-phenylethyl)pentanamide
44. (2S)-2-(adamantan-1-ylformamido)-5-(4-benzylpiperidin-1-yl)-N-(2-phenylethyl)pentanamide
45. (2S)-5-(4-benzylpiperidin-1-yl)-2-(3,3-diphenylprop-2-enamido)-N-(2-phenylethyl)pentanamide
46. (2S)-5-(4-benzylpiperidin-1-yl)-N-(2-phenylethyl)-2-[4-(phenylformamido)butanamido]pentanamide; trifluoroacetic acid
47. N-(2-phenylethyl)-2-(phenylformamido)-5-(piperidin-1-yl)pentanamide
48. (2S)-N-[2-(1H-indol-3-yl)ethyl]-2-[(2-phenylphenyl)formamido]-5-(piperidin-1-yl)pentanamide
49. N-[(2S)-1-oxo-5-(piperidin-1-yl)-1-(1,2,3,4-tetrahydroisoquinolin-2-yl)pentan-2-yl]-2-phenylbenzamide
50. (2S)-N-[3-(4-chlorophenyl)propyl]-2-[(2-phenylphenyl)formamido]-5-(piperidin-1-yl)pentanamide
51. (2S)-N-[2-(3,4-dichlorophenyl)ethyl]-2-[(2-phenylphenyl)formamido]-5-(piperidin-1-yl)pentanamide
52. N-[(2S)-1-oxo-5-(piperidin-1-yl)-1-(2,3,4,5-tetrahydro-1H-2-benzazepin-2-yl)pentan-2-yl]-2-phenylbenzamide
53. (2S)-2-[(2-phenylphenyl)formamido]-5-(piperidin-1-yl)-N-{2-[4-(trifluoromethyl)phenyl]ethyl}pentanamide
54. (2S)-2-[(2-phenylphenyl)formamido]-N-(2-phenylpropyl)-5-(piperidin-1-yl)pentanamide
55. (2S)-2-(1-benzofuran-2-ylformamido)-N-(2-phenylethyl)-5-(piperidin-1-yl)pentanamide
56. (2S)-2-(1H-indol-2-ylformamido)-N-(2-phenylethyl)-5-(piperidin-1-yl)pentanamide
57. (2S)-N-(2-phenylethyl)-2-[(2E)-3-phenylprop-2-enamido]-5-(piperidin-1-yl)pentanamide
58. (2S)-2-[3-(1H-indol-3-yl)propanamido]-N-(2-phenylethyl)-5-(piperidin-1-yl)pentanamide
59. (2S)-2-[3-(10H-phenothiazin-10-yl)propanamido]-N-(2-phenylethyl)-5-(piperidin-1-yl)pentanamide
60. (2S)-N-(2-phenylethyl)-5-(piperidin-1-yl)-2-{9H-pyrido[3,4-b]indol-3-ylformamido}pentanamide
61. (2S)-2-{imidazo[1,2-a]pyridin-2-ylformamido}-N-(2-phenylethyl)-5-(piperidin-1-yl)pentanamide
62. N-[(2S)-1-oxo-5-(piperidin-1-yl)-1-(1,2,3,4-tetrahydroisoquinolin-2-yl)pentan-2-yl]-1-benzofuran-2-carboxamide
63. (2S)-2-(1-benzofuran-2-ylformamido)-N-[3-(4-chlorophenyl)propyl]-5-(piperidin-1-yl)pentanamide
64. (2S)-2-(1-benzofuran-2-ylformamido)-N-[(2R)-1-phenylpropan-2-yl]-5-(piperidin-1-yl)pentanamide
65. N-[(2S)-1-oxo-5-(piperidin-1-yl)-1-(1,2,3,4-tetrahydroisoquinolin-2-yl)pentan-2-yl]-3-(10H-phenothiazin-10-yl)propanamide
66. (2S)-N-[3-(4-chlorophenyl)propyl]-2-[3-(10H-phenothiazin-10-yl)propanamido]-5-(piperidin-1-yl)pentanamide
67. (2S)-2-[3-(10H-phenothiazin-10-yl)propanamido]-N-[(2R)-1-phenylpropan-2-yl]-5-(piperidin-1-yl)pentanamide
68. (2E)-N-[(2S)-1-oxo-5-(piperidin-1-yl)-1-(1,2,3,4-tetrahydroisoquinolin-2-yl)pentan-2-yl]-3-phenylprop-2-enamide
69. (2S)-N-[3-(4-chlorophenyl)propyl]-2-[(2E)-3-phenylprop-2-enamido]-5-(piperidin-1-yl)pentanamide
70. (2S)-2-[(2E)-3-phenylprop-2-enamido]-N-[(2R)-1-phenylpropan-2-yl]-5-(piperidin-1-yl)pentanamide.

7. A pharmaceutical composition comprising a compound of formula (I) as defined in any one of claims 1-6 in a pharmaceutically acceptable support.

8. A compound according to anyone of claims 1-6 or a composition according to claim 7 for use in a therapeutic method, in particular for the treatment of pain.

9. The compound or composition for use according to claim 8, to decrease or block hyperalgesia and/or tolerance effects related to the use of an analgesic compound, in particular an opiate analgesic compound, or to decrease or block exaggerated hypersensitivity to a future nociceptive stimulus.

10. The compound or composition for use according to claim 9, wherein the analgesic compound is selected from morphine, fentanyl, sufentanil, alfentanyl, heroin, oxycodone, hydromorphone, levorphanol, methadone, buprenorphine, butorphanol, meperidine and a mixture therof,.

11. The compound or composition for use according to claim 8, 9 or 10, for the treatment of hyperalgesia occurring or associated with acute or chronic pain in response to surgery, trauma or pathology of a mammal.

12. The compound or composition for use according to anyone of claims 8-11, wherein the compound is administered by a systemic route, more specifically by oral route.

13. The compound or composition for use according to anyone of claims 8-12, for the treatment of patients suffering from cancer or burns.

14. The compound or composition for use according to anyone of claims 8-12, for the treatment of postoperative pain.

15. The compound or composition for use according to anyone of claims 8-14 , wherein the compound is administered simultaneously with, separately from or sequentially to an analgesic compound.
